(19) Europäisches Patentamt
European Patent Office
Office européen des brevets

(11) Veröffentlichungsnummer: **0 013 905**
**B1**

(12) **EUROPÄISCHE PATENTSCHRIFT**

(45) Veröffentlichungstag der Patentschrift: **05.05.82**

(51) Int. Cl.³: **C 07 D 233/68,** A 01 N 43/50

(21) Anmeldenummer: **80100105.8**

(22) Anmeldetag: **10.01.80**

(54) **4,5-Dichlorimidazol-Derivate, Verfahren zu ihrer Herstellung sowie ihre Verwendung als Herbizide.**

(30) Priorität: **18.01.79 DE 2901862**

(43) Veröffentlichungstag der Anmeldung: **06.08.80 Patentblatt 80/16**

(45) Bekanntmachung des Hinweises auf die Patenterteilung: **05.05.82 Patentblatt 82/18**

(84) Benannte Vertragsstaaten: **AT BE CH DE FR GB IT NL SE**

(56) Entgegenhaltungen:
**DE-A-1 567 084**
**DE-A-1 926 206**
**DE-A-2 610 527**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Beck, Gunther, Dr., Am Mittelberg 19, D-5090 Leverkusen 1 (DE)**
Erfinder: **Heitzer, Helmut, Dr., Höhenstrasse 84, D-5090 Leverkusen 3 (DE)**
Erfinder: **Eue, Ludwig, Dr., Paul-Klee-Strasse 36, D-5090 Leverkusen 1 (DE)**
Erfinder: **Schmidt, Robert Rudolf, Dr., Hahnenweg 5, D-5000 Köln 80 (DE)**

**EP 0 013 905 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 4,5-Dichlorimidazolderivate, Verfahren zu ihrer Herstellung sowie ihre Verwendung als Herbizide

Die Erfindung betrifft neue 4,5-Dichlorimidazolderivate, mehrere Verfahren zu ihrer Herstellung sowie ihre Verwendung als Herbizide.

Es ist bereits bekannt geworden, dass bestimmte 4,5-Dichlorimidazol-2-carbonsäurederivate herbizide Eigenschaften besitzen (vgl. DE-OS Nr. 2610527). So kann zum Beispiel das 4,5-Dichlorimidazol-2-carbonsäureethylamid zur Bekämpfung von Unkraut eingesetzt werden. Dieser Stoff ist jedoch nicht immer ausreichend wirksam und in seiner Selektivität nicht immer ganz befriedigend.

Es wurden nun neue 4,5-Dichlorimidazolderivate der Formel I

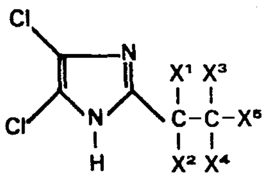

(I)

in welcher

$X^1$ für Wasserstoff, Chlor, Brom oder Jod steht,

$X^2$, $X^3$ und $X^4$ jeweils für Wasserstoff, Chlor oder Brom stehen,

$X^5$ für Wasserstoff oder Chlor steht, und ausserdem

$X^1$ und $X^3$ gemeinsam für eine C-C-Bindung stehen können und auch

$X^2$ und $X^4$ gemeinsam für eine C-C-Bindung stehen können, oder

$X^1$ und $X^2$ gemeinsam für Sauerstoff oder die Gruppierung $=N-Y$ stehen, in welcher

Y für Hydroxy und die Reste

$-NH-R^1$ oder 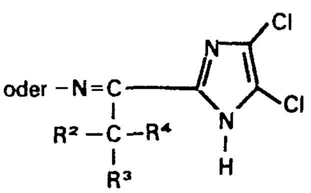 steht,

in welchen

R für Wasserstoff oder Alkyl mit 1 bis 4 Kohlenstoffatomen steht,

$R^1$ für gegebenenfalls durch Fluor, Chlor, Brom, Alkyl mit 1 oder 2 Kohlenstoffatomen, Trichlormethyl, Trifluormethyl und/oder Nitro substituiertes Phenyl oder Naphthyl steht,

Z für Sauerstoff oder Schwefel steht, und

$R^2$, $R^3$ und $R^4$ unabhängig voneinander für Wasserstoff oder Chlor stehen,

wobei jedoch $X^1$, $X^2$, $X^3$, $X^4$ und $X^5$ nicht gleichzeitig für Wasserstoff stehen,

gefunden.

Weiterhin wurde gefunden, dass man 4,5-Dichlorimidazolderivate der Formel I erhält, wenn man

a) 2-Ethylimidazol der Formel II

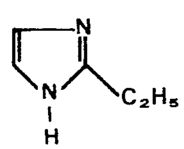

(II)

oder dessen Hydrochlorid mit bis zu 7 mol Chlor/mol an 2-Ethylimidazol in einem unter den Reaktionsbedingungen inerten Lösungsmittel umsetzt, oder

b) ein 4,5-Dichlorimidazolderivat der Formel III

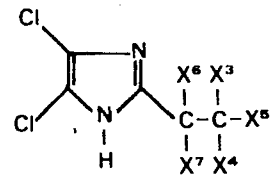

(III)

in welcher

$X^6$ für Chlor oder Brom steht,

$X^7$ für Chlor oder Brom steht, und

$X^3$, $X^4$ und $X^5$ die oben angegebene Bedeutung haben, wobei jedoch $X^3$, $X^4$ und $X^5$ nicht gleichzeitig für Wasserstoff stehen,

α) mit Wasser hydrolysiert,

oder

β) mit mindestens der stöchiometrisch erforderlichen Menge eines Dehalogenierungsmittel gegebenenfalls in Gegenwart eines Verdünnungsmittels umsetzt,

oder

γ) mit mindestens der stöchiometrisch erforderlichen Menge eines zur Hydrierung gebräuchlichen Metallhydrids in Gegenwart eines Verdünnungsmittels umsetzt,

oder

c) ein 4,5-Dichlorimidazolderivat der Formel IV

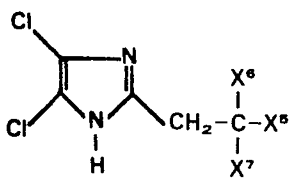

(IV)

in welcher:

$X^5$, $X^6$ und $X^7$ die oben angegebene Bedeutung haben,

mit einem Chlorwasserstoff abspaltenden Mittel in Gegenwart eines Verdünnungsmittels umsetzt,

oder

d) das 2,4,5-Trichlor-2-1(1,1-dichlorethyl)-2 H-imidazol der Formel V

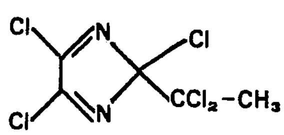

(V)

α) mit einem Reduktionsmittel in Gegenwart eines Verdünnungsmittels umsetzt,

oder

β) mit einem mindestens 3-molaren Überschuss eines Alkalimetalljodids oder mit einem mindestens 1,5-molaren Überschuss eines Erdalkalimetalljodids in Gegenwart eines niederen aliphatischen Ketons als Verdünnungsmittel umsetzt,

oder

χ) mit einer mindestens äquimolaren Menge eines Alkalimetallbromids oder mit einer mindestens äquivalenten Menge eines Erdakalimetallbromids in Gegenwart eines niederen aliphatischen Nitrils oder eines niederen aliphatischen Ketons als Verdünnungsmittel umsetzt,

oder

e) ein 4,5-Dichlorimifazolderivat der Formel VI

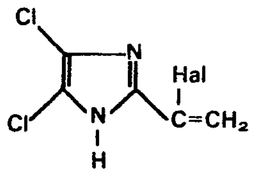

(VI)

in welcher

Hal für Chlor oder Jod steht,

α) mit einem Halogenwasserstoff abspaltenden Mittel gegebenenfalls in Gegenwart eines Verdünnungsmittels umsetzt,

oder

β) mit Wasser in Gegenwart einer Base oder eines basischen Salzes hydrolysiert,

oder

f) ein 4,5-Dichlorimidazolderivat der Formel VII

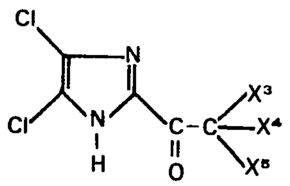

(VII)

in welcher

$X^3$, $X^4$ und $X^5$ die oben angegebene Bedeutung haben,

mit einer Verbindung der Formel VIII

$$H_2N-Y' \qquad (VIII)$$

in welcher

Y' für Amino, Hydroxy und die Reste

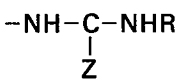

oder $-NHR^1$ steht

in welchen

Z für Sauerstoff oder Schwefel steht,

R für Wasserstoff oder Alkyl mit 1 bis 4 Kohlenstoffatomen steht, und

$R^1$ für gegebenenfalls durch Fluor, Chlor, Brom, Alkyl mit 1 oder 2 Kohlenstoffatomen, Alkoxy mit 1 oder 2 Kohlenstoffatomen, Trichlormethyl, Trifluormethyl und/oder Nitro substituiertes Phenyl oder Naphthyl steht,

in Gegenwart eines Verdünnungsmittels umsetzt,

oder

g) das 4,5-Dichlor-2-ethinylimidazol der Formel IX

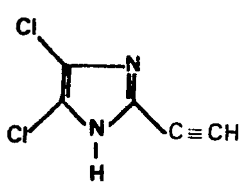

(IX)

mit einem Chlorierungs- oder Bromierungsmittel in Gegenwart eines unter den Reaktionsbedingungen inerten Lösungsmittels umsetzt,

oder

h) 4,5-Dichlorimidazolderivate der Formel X

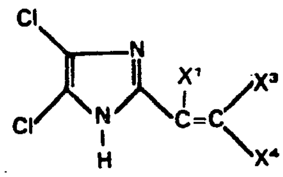

(X)

in welcher

$X^1$, $X^3$ und $X^4$ die oben angegebene Bedeutung haben, mit einem Chlorierungs- oder Bromierungsmittel in Gegenwart eines unter den Reaktionsbedingungen inerten Lösungsmittels umsetzt,

oder

i) 4,5-Dichlorimidazolderivate der Formel XI

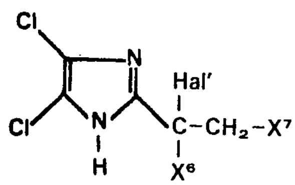

(XI)

in welcher

$X^6$ und $X^7$ die oben angegebene Bedeütung haben und

Hal' für Chlor, Brom oder Jod steht,

in Gegenwart eines niederen aliphatischen Nitrils thermisch dehydrohalogeniert.

Schliesslich wurde gefunden, dass die neuen 4,5-Dichlorimidazolderivate der Formel I starke herbizide, insbesondere auch selektiv-herbizide Eigenschaften besitzen.

Überraschenderweise zeigen die erfindungsgemässen 4,5-Dichlorimidazolderivate der Formel I bei sehr guter Unkrautwirkung insbesondere bessere Möglichkeiten zum Einsatz als selektive Unkrautbekämpfungsmittel in wichtigen Kulturen als das aus dem Stand der Technik bekannte 4,5-Dichlorimidazol-2-carbonsäureethylamid, welches ein hochaktiver Wirkstoff gleicher Wirkungsart ist. Die erfindungsgemässen Stoffe stellen somit eine wertvolle Bereicherung der herbiziden Mittel zur selektiven Unkrautbekämpfung dar.

Die erfindungsgemässen 4,5-Dichlorimidazolderivate sind durch die Formel I allgemein definiert. In dieser Formel haben $X^1$, $X^2$, $X^3$, $X^4$ und $X^5$ die oben angegebene Bedeutung.

In den für Y angegebenen Resten steht R vorzugsweise für Wasserstoff oder Alkyl mit 1 bis 2 Kohlenstoffatomen, insbesondere jedoch für Wasserstoff. Z steht vorzugsweise für Schwefel. $R^1$ steht für die oben angegebenen Reste und $R^2$, $R^3$ und $R^4$ stehen vorzugsweise für Wasserstoff.

Verwendet man bei dem erfindungsgemässen Verfahren (a) auf 1 mol 2-Ethylimidazol 7 mol Chlor, so kann der Verlauf der Umsetzung durch das folgende Formelschema wiedergegeben werden:

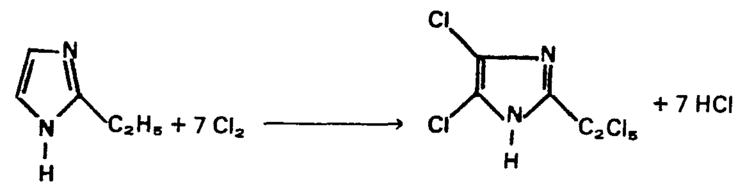

Verwendet man 4,5-Dichlor-2-pentachlorethyl-imidazol als Ausgangsstoff und Wasser als Hydrolysierungsmittel, so kann der Verlauf des erfindungsgemässen Verfahrens (b), Variante α, durch das folgende Formelschema wiedergegen werden:

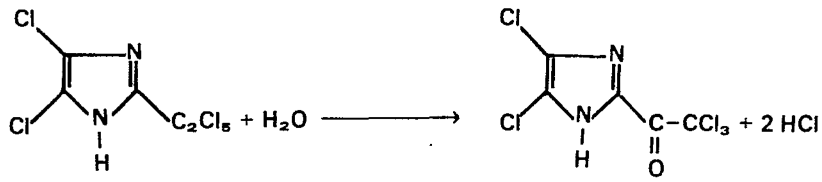

Verwendet man 4,5-Dichlor-2-pentachlorethyl-imidazol als Ausgangsstoff und Natriumjodid in Aceton als Dechlorierungsmittel, so kann der Verlauf des erfindungsgemässen Verfahrens (b), Variante β, durch das folgende Formelschema wiedergegeben werden:

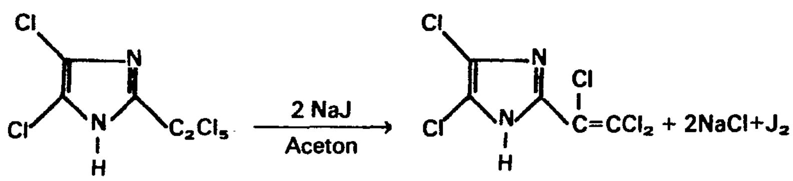


Verwendet man 4,5-Dichlor-2-pentachlorethyl-imidazol als Ausgangsstoff und Natriumboranat als Hydrierungsmittel, so kann der Verlauf des erfindungsgemässen Verfahren (b), Variante γ, durch das folgende Formelschema wiedergegeben werden:

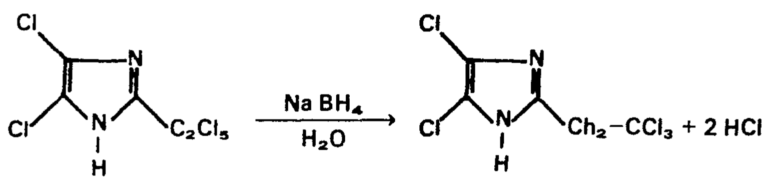


Verwendet man 4,5-Dichlor-2-(2,2,2-trichlor-ethyl)imidazol als Ausgangsstoff und Triethylamin als Chlorwasserstoff abspaltendes Mittel, so kann der Verlauf des erfindungsgemässen Verfahrens (c) durch das folgende Formelschema wiedergegeben werden:

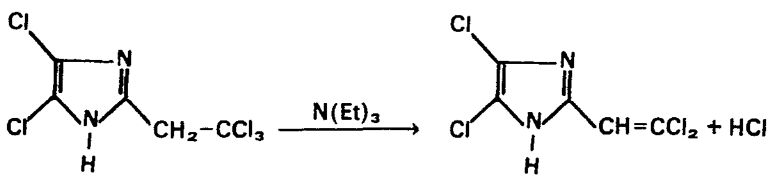


Verwendet man das 2,4,5-Trichlor-2-(1,1-dichlorethyl)-2H-imidazol als Ausgangsstoff und katalytisch aktivierten Wasserstoff als Reduktionsmittel, so kann der Verlauf des erfindungsgemässen Verfahrens (d), Variante α, durch das folgende Formelschema wiedergegeben werden:

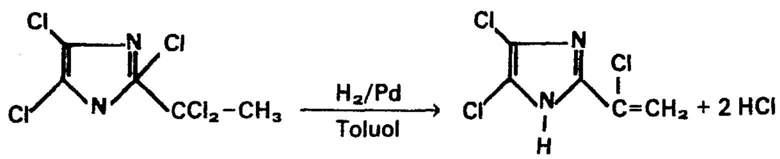


Verwendet man das 2,4,5-Trichlor-2-(1,1-dichlorethyl)-2H-imidazol und Natriumjodid als Ausgangsstoffe, so kann der Verlauf des erfindungsgemässen Verfahrens (d), Variante β, durch das folgende Formelschema wiedergegeben werden:

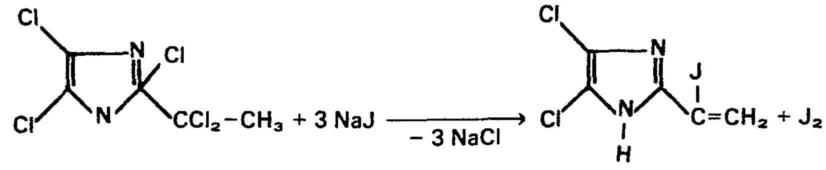

Verwendet man das 2,4,5-Trichlor-2-(1,1-dichlorethyl)-2H-imidazol und Kaliumbromid als Ausgangsstoffe, so kann der Verlauf des erfindungsgemässen Verfahren (d), Variante γ, durch das folgende Formelschema wiedergegeben werden:

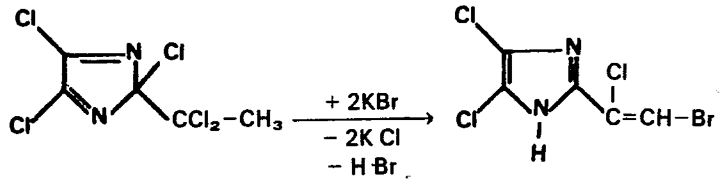


Verwendet man 4,5-Dichlor-2-(1-jodethinyl)-imidazol als Ausgangsstoff und Triethylamin als Halogenwasserstoff abspaltendes Mittel, so kann der Verlauf des erfindungsgemässen Verfahrens (e), Variante α, durch das folgende Formelschema wiedergegeben werden:

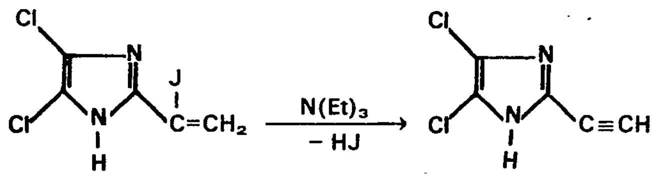


Verwendet man 4,5-Dichlor-2-(1-jodvinyl)-imidazol als Ausgangsstoff und Wasser sowie Natriumformiat als Hydrolysierungsmittel, so kann der Verlauf des erfindungsgemässen Verfahrens (e), Variante β, durch das folgende Formelschema wiedergegeben werden:

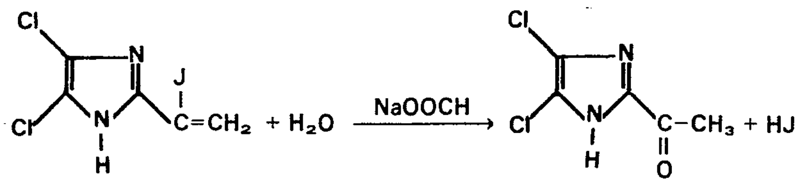


Verwendet man 4,5-Dichlor-2-acetylimidazol und Phenylhydrazin als Ausgangssubstanzen, so kann der Verlauf des erfindungsgemässen Verfahrens (f) durch das folgende Formelschema wiedergegeben werden:

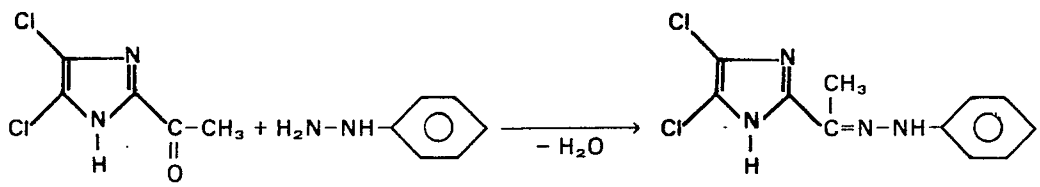


Verwendet man 4,5-Dichlor-2-ethylimidazol als Ausgangssubstanz und Sulfurylchlorid als Chlorierungsmittel, so kann der Verlauf des erfindungsgemässen Verfahrens (g) durch das folgenden Formelschema wiedergegeben werden:

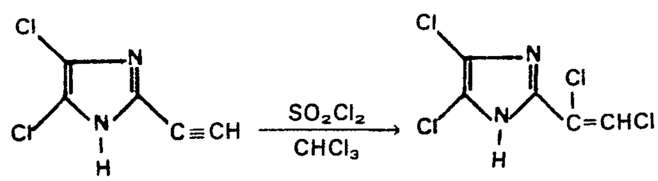


Verwendet man 4,5-Dichlor-2-(1-chlorvinyl)-imidazol als Ausgangssubstanz und Sulfurylchlorid als Chlorierungsmittel, so kann der Verlauf des erfindungsgemässen Verfahrens (h) durch das folgende Formelschema wiedergegeben werden:

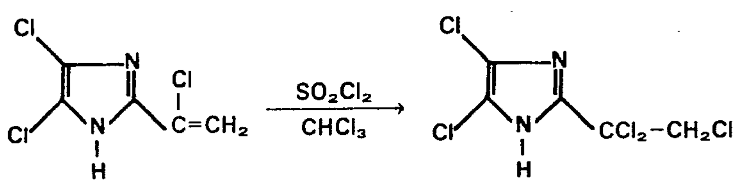

Verwendet man 4,5-Dichlor-2-(1,1,2-trichlorethyl)imidazol als Ausgangsstoff, so kann der Verlauf des erfindungsgemässen Verfahrens (i) durch das folgende Formelschema wiedergegeben werden:

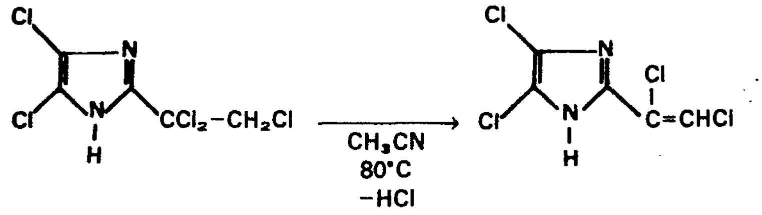


Das bei dem erfindungsgemässen Verfahren (a) als Ausgangsprodukt benötigte 2-Ethylimidazol bzw. dessen Hydrochlorid ist bekannt.

Als Chlorierungsmittel wird bei dem erfindungsgemässen Verfahren (a) Chlorgas verwendet.

Als Lösungsmittel kommen bei der Durchführung des erfindungsgemässen Verfahrens (a) alle üblichen unter den Reaktionsbedingungen inerten Solventien in Frage.

Hierzu gehören vorzugsweise Phosphoroxidchlorid, 1,1,2,2-Tetrachlorethan, Tetrachlorethylen und Thionylchlorid.

Die Reaktionstemperaturen können bei dem erfindungsgemässen Verfahren (a) je nach dem gewünschten Endprodukt innerhalb eines bestimmten Bereiches variiert werden. Soll das 4,5-Dichlor-2-pentachlorethylimidazol synthetisiert werden, so arbeitet man im allgemeinen bei Temperaturen zwischen 90 und 150°C, vorzugsweise zwischen 100 und 130°C. Zur Herstellung niedriger chlorierter Verbindungen arbeitet man im allgemeinen bei Temperaturen zwischen 60 und 100°C, vorzugsweise zwischen 70 und 90°C.

Bei der Durchführung des erfindungsgemässen Verfahrens (a) setzt man das 2-Ethylimidazol bzw. dessen Hydrochlorid je nach dem gewünschten Endprodukt mit unterschiedlichen Mengen an Chlor um. Soll das 4,5-Dichlor-2-pentachlorethylimidazol hergestellt werden, so setzt man auf 1 mol an 2-Ethylimidazol etwa 3 bis 7 mol Chlor ein. Zur Abmilderung der stark exothermen Chlorierungsreaktion kann man die ebenfalls exotherme Hydrochloridbildung aus 2-Ethylimidazol und Chlorwasserstoff dadurch ausschalten, dass man nicht das 2-Ethylimidazol selbst, sondern dessen Hydrochlorid als Ausgangsprodukt verwendet. Die Isolierung des Reaktionsproduktes erfolgt nach üblichen Methoden. Im allgemeinen verfährt man in der Weise, dass man das Lösungsmittel abdestilliert und den verbleibenden Rückstand mit Ameisensäure bei Raumtemperatur verrührt. Das dabei kristallin anfallende Reaktionsprodukt wird abfiltriert. Man kann aber auch aus dem vom Lösungsmittel befreiten Rückstand das Reaktionsprodukt im Vakuum, z.B. bei 125°C/0,01 Torr, heraussublimieren.

Soll bei dem Verfahren (a) ein 4,5-Dichlorimidazolderivat der Formel I hergestellt werden, in dem die Seitenkette weniger als 5 Chloratome enthält, so setzt man auf 1 mol an 2-Ethylimidazol oder dessen Hydrochlorid bis zu 6 mol Chlor ein, hält dabei aber die Reaktionstemperatur niedriger als bei der Perchlorierung der Ethylgruppe. Das Reaktionsprodukt entsteht manchmal nicht in reiner Form, sondern im Gemisch mit wechselnden Mengen an 4,5-Dichlor-2-pentachlorethylimidazol. Das gewünschte Reaktionsprodukt kann dann jedoch durch übliche Trennmethoden, wie (fraktionierte) Kristallisation, z.B. aus Chlorbenzol oder aus Aceton, oder durch Chromatographie rein gewonnen werden.

Die bei der Durchführung des erfindungsgemässen Verfahrens (b) als Ausgangsstoffe benötigten 4,5-Dichlorimidazolderivate sind durch die Formel III definiert.

Die Verbindungen der Formel III sind bisher noch nicht bekannt. Sie lassen sich jedoch nach dem erfindungsgemässen Verfahren (a) bzw. nach anderen der erfindungsgemässen Verfahren herstellen.

Bei der Durchführung der Variante (α) des erfindungsgemässen Verfahrens (b) wird ein 4,5-Dichlorimidazolderivat der Formel III mit Wasser hydrolysiert. Das Wasser fungiert hierbei gleichzeitig als Reaktionsmedium und wird deshalb in grossem Überschuss eingesetzt.

Die Reaktionstemperatur kann bei der Durchführung der Variante (α) des erfindungsgemässen Verfahrens (b) in einem bestimmten Bereich variiert werden. Im allgemeinen arbeitet man bei Temperaturen zwischen 70 und 120°C, vorzugsweise bei der Siedetemperatur des Reaktionsgemisches.

Die Isolierung der Reaktionsprodukte erfolgt bei der Umsetzung nach Variante α des erfindungsgemässen Verfahrens (b) nach üblichen Methoden. Im allgemeinen fallen die Reaktionsprodukte kristallin an, so dass sie abfiltriert werden können.

Bei der Durchführung der Variante β des erfindungsgemässen Verfahrens (b) kommen als Dehalogenierungsmittel alle üblicherweise für derartige Zwecke einsetzbaren Reagenzien in Frage. Vorzugsweise verwendbare Dehalogenierungsmittel sind hierbei Natriumjodid in Aceton, Kupfer-(I)-chlorid, Zinkstaub, Eisenpulver, Schwefel und Phosphor.

Als Verdünnungsmittel kommen bei der Variante β des erfindungsgemässen Verfahrens (b) alle für derartige Dehalogenierungsreaktionen üblichen Solventien in Betracht. Vorzugsweise verwendbar sind Aceton, Toluol, Chlorbenzol, 1,2-Dichlorbenzol und 1,2,4-Trichlorbenzol.

Besonders bevorzugt als Dehalogenierungsmittel ist das System Natriumjodid/Aceton.

Die Reationstemperaturen können bei der Variante β des erfindungsgemässen Verfahrens (b) in

einem grösseren Bereich variiert werden. Im allgemeinen arbeitet man bei Temperaturen zwischen 0 und 200°C, vorzugsweise zwischen 50 und 150°C. Bei Verwendung des Systems Natriumjodid/Aceton arbeitet man im allgemeinen bei Temperaturen zwischen 0 und 50°C, vorzugsweise zwischen 20 und 30°C.

Bei der Durchführung der Variante β des erfindungsgemässen Verfahrens (b) setzt man das 4,5-Dichlorimidazolderivat der Formel III mit mindestens der stöchiometrischen Menge an Dehalogenierungsmittel um. Verwendet man Natriumjodid, so kann dies in einem mehrfachen Überschuss eingesetzt werden, ohne dass eine weitere Dechlorierung erfolgt. Die Isolierung der Reaktionsprodukte erfolgt nach üblichen Methoden. Im allgemeinen verfährt man bei Verwendung von Natriumjodid als Dechlorierungsmittel in der Weise, dass man das Reaktionsgemisch in Wasser giesst, das freie Jod durch Zusatz eines Reduktionsmittels, wie z.B. wässriger Natriumhydrogensulfitlösung, entfernt und dann das kristallin anfallende Produkt absaugt.

Bei der Durchführung der Variante γ des erfindungsgemässen Verfahrens (b) kommen als Hydrierungsmittel alle für derartige Umsetzungen üblicherweise gebräuchlichen (komplexen) Metallhydride in Frage. Speziell genannt sei Natriumboranat.

Als Verdünnungsmittel kommen bei der Variante γ des erfindungsgemässen Verfahrens (b) alle für derartige Umsetzungen üblicherweise verwendbaren Lösungsmittel in Betracht. Speziell genannt sei Wasser.

Besonders bevorzugt ist das System Natriumboranat/Wasser.

Die Reationstemperaturen können bei der Variante γ des erfindungsgemässen Verfahrens in einem grösseren Bereicht variiert werden. Im allgemeinen arbeitet man bei Temperaturen zwischen 0 und 50°C, vorzugsweise zwischen 10 und 40°C. Bei Verwendung des Systems Natriumboranat/Wasser arbeitet man bevorzugt bei Raumtemperatur oder wenig darunter.

Bei der Durchführung der Variante γ des erfindungsgemässen Verfahrens (b) setzt man das 4,5-Dichlorimidazolderivat der Formel III mit mindestens der stöchiometrisch erforderlichen Menge an Metallhydrid um. Verwendet man Natriumsboranat, so kann dies in einem mehrfachen, z.B. bis zu zehnfachen, Überschuss eingesetzt werden, ohne dass weitere unerwünschte Hydrierungen erfolgen. Die Isolierung der Reaktionsprodukte erfolgt nach üblichen Methoden. Bei Verwendung von Natriumboranat als Hydrierungsmittel verfährt man im allgemeinen in der Weise, dass man zunächst gegebenenfalls vorhandenes überschüssiges Natriumboranat durch vorsichtigen Säurezusatz zerstört und gegebenenfalls anfallende Borsäure mit heissem Wasser aus dem Reaktionsgemisch herauslöst.

Die bei der Durchführung des erfindungsgemässen Verfahrens (c) als Ausgangsstoffe benötigten 4,5-Dichlorimidazolderivate sind durch die Formel IV definiert.

Die Verbindungen der Formel IV sind bisher noch nicht bekannt; sie lassen sich jedoch nach dem erfindungsgemässen Verfahren (b), Variante γ, herstellen.

Bei der Durchführung des Verfahrens (c) kommen als Halogenwasserstoff abspaltende Mittel alle üblicherweise für derartige Umsetzungen gebräuchlichen Reagenzien in Frage. Hierzu gehören anorganische und organische Basen, wie Alkalimetall- und Erdalkalimetallhydroxide bzw. -carbonate und -hydrogencarbonate, ferner Aluminiumoxide und organische Amine, insbesondere tertiäre Amine, wie z.B. Triethylamin.

Als Verdünnungsmittel kommen bei dem erfingungsgemässen Verfahren (c) alle für derartige Dehydrohalogenierungsreaktionen üblichen Solventien in Betracht.

Vorzugsweise verwendbar ist Wasser oder ein Amin, das gleichzeitig als Halogenwasserstoff abspaltendes Mittel dient, sowie Dioxan.

Die Reaktionstemperaturen können bei dem erfindungsgemässen Verfahren (c) innerhalb eines grösseren Bereiches variiert werden. Im allgemeinen arbeitet man bei Temperaturen zwischen 0 und 150°C, vorzugsweise zwischen 20 und 120°C.

Bei der Durchführung des erfindungsgemässen Verfahrens (c) setzt man im allgemeinen auf 1 mol an 4,5-Dichlorimidazolderivate der Formel IV 1 bis 1,2 mol an Dehydrohalogenierungsmittel ein. Die Isolierung der Reaktionsprodukte erfolgt nach üblichen Methoden. Im allgemeinen geht man so vor, dass man das Reaktionsgemisch ansäuert, gegebenenfalls mit Wasser versetzt und anschliessend das kristallin anfallende Reaktionprodukt abfiltriert.

Das bei dem erfindungsgemässen Verfahren (d) als Ausgangsstoff benötigte 2,4,5-Trichlor-2-(1,1-dichlorethyl)-2H-imidazol der Formel V ist bekannt (vgl. DE-OS Nr. 2550157).

Als Reduktionsmittel kommen bei der Variante α des erfindungsgemässen Verfahrens (d) vorzugsweise durch Edelmetalle, wie z.B. Platin oder Palladium, angeregter Wasserstoff oder auch Reduktionsmittel, wie Jodwasserstoff oder Bromwasserstoff, gegebenenfalls in Gegenwart von Schwefeldioxid in Frage.

Als Verdünnungsmittel können bei der Variante α des erfindungsgemässen Verfahrens (d) je nach dem eingesetzten Reduktionsmittel verschiedene Solventien verwendet werden. So werden bei der katalytischen Hydrierung vorzugsweise aromatische Kohlenwasserstoffe, wie Benzol, Toluol und Xylol, ferner cyclische Äther, wie Dioxan und Tetrahydrofuran, als Verdünnungsmittel eingesetzt. Führt man die Umsetzung mit chemischen Stoffen, wie Bromwasserstoff oder Jodwasserstoff, durch, so kommen als Lösungsmittel vorzugsweise inerte, mit Wasser nicht mischbare organische Solventien, wie Methylenchlorid, Chloroform oder Toluol, in Betracht.

Die Reaktionstemperaturen können bei der Variante α des erfindungsgemässen Verfahrens (d) innerhalb eines bestimmten Bereiches variiert werden. Im allgemeinen arbeitet man bei Temperaturen zwischen 0 und 50°C, vorzugsweise zwischen 10 und 30°C.

Bei der Durchführung der Variante α des erfindungsgemässen Verfahrens (d) setzt man auf 1 mol 2,4,5-Trichlor-2-(1,1-dichlorethyl)-2H-imidazol der Formel V entweder einen Überschuss an katalytisch angeregtem Wasserstoff oder mindestens 2 mol eines chemischen Reduktionsmittels, wie Bromwasserstoff oder Jodwasserstoff, ein. Bromwasserstoff und Jodwasserstoff können vorzugsweise in Form ihrer handelsüblichen konzentrierten wässrigen Lösungen (Azeotrope) eingesetzt werden. Hierbei wirkt es sich günstig auf die Ausbeute an Endprodukt aus, wenn man die Reaktionsmischungen mit Schwefeldioxidgas sättigt.

Die Isolierung des Endprodukts erfolgt nach üblichen Methoden. Im allgemeinen verfährt man in der Weise, dass man zunächst das Lösungsmittel abzieht, den verbleibenden Rückstand in Wasser suspendiert, dann neutralisiert und absaugt. Das Produkt kann durch Umkristallisation, z.B. aus Acetonitril, oder durch Vakuumsublimation gereinigt werden.

Als Reaktionskomponenten kommen bei der Variante β des erfindungsgemässen Verfahrens (d) Alkalimetalljodide und Erdalkalimetalljodide in Frage. Hierzu gehören vorzugsweise Natriumjodid, Kaliumjodid und Calciumjodid.

Als Verdünnungsmittel können bei der Variante β des erfindungsgemässen Verfahrens (d) alle üblichen niederen aliphatischen Ketone verwendet werden. Besonders bevorzugt ist Aceton.

Die Reaktionstemperaturen können bei der Variante β des erfindungsgemässen Verfahrens (d) innerhalb eines grösseren Bereiches variiert werden. Im allgemeinen arbeitet man bei Temperaturen zwischen −20 und +100°C vorzugsweise zwischen 0 und 60°C.

Bei der Durchführung der Variante β des erfindungsgemässen Verfahrens (d) setzt man auf 1 mol an 2,4,5-Trichlor-2-(1,1-dichlorethyl)-2H-imidazol der Formel V mindestens 3 mol an Alkalimetalljodid bzw. mindestens 1,5 mol an Erdalkalimetalljodid ein. Besonders bevorzugt ist das System Natriumjodid/Aceton. Hierbei kann das Natriumjodid in einem grossen Überschuss verwendet werden, ohne dass störende Nebenreaktionen ablaufen. Die Isolierung des Reaktionsproduktes erfolgt nach üblichen Methoden. Im allgemeinen verfährt man in der Weise, dass man das Reaktionsgemisch in Wasser eingiesst, durch Zugabe eines Reduktionsmittels, wie wässrige Natriumhydrogensulfitlösung, das vorhandene freie Jod entfernt und anschliessend filtriert.

Als Reaktionskomponenten kommen bei der Variante γ des erfindungsgemässen Verfahrens (d) Alkalimetallbromide und Erdalkalimetallbromide in Frage. Hierzu gehören vorzugsweise Natriumbromid, Kaliumbromid und Calciumbromid. Besonders bevorzugt ist Kaliumbromid.

Als Verdünnungsmittel können bei der Variante γ des erfindungsgemässen Verfahrens (d) alle üblichen niederen aliphatischen Nitrile bzw. niederen aliphatischen Ketone verwendet werden. Besonders bevorzugt sind Acetonitril bzw. Aceton.

Die Reaktionstemperaturen können bei der Variante γ des erfindungsgemässen Verfahrens (d) innerhalb eines grösseren Bereiches variiert werden. Im allgemeinen arbeitet man bei Temperaturen zwischen 50 und 150°C, vorzugsweise zwischen 70 und 120°C.

Bei der Durchführung der Variante γ des erfindungsgemässen Verfahrens (d) setzt man auf 1 mol an 2,4,5-Trichlor-2-(1,1-dichlorethyl)-2H-imidazol der Formel V mindestens 1 mol an Alkalimetallbromid bzw. mindestens 0,5 mol an Erdalkalimetallbromid ein. Besonders bevorzugt sind die Systeme Kaliumbromid/Acetonitril und Natriumbromid/Aceton. Hierbei verwendet man das Alkalimetallbromid vorzugsweise in einem 5- bis 10fach molaren Überschuss. Die Isolierung der Reaktionsprodukte erfolgt nach üblichen Methoden. Im allgemeinen verfährt man in der Weise, dass man nach dem Erkalten in Wasser giesst (5- bis 10fache Menge), die wässrige Suspension filtriert, den Rückstand mit Wasser wäscht und trocknet.

Die bei dem erfindungsgemässen Verfahren (e) als Ausgangsstoffe benötigten 4,5-Dichlorimidazolderivate sind durch die Formel VI definiert.

Die Verbindungen der Formel VI sind bisher noch nicht bekannt. Sie lassen sich jedoch nach dem erfindungsgemässen Verfahren (d), Varianten α und β herstellen.

Als Dehydrohalogenierungsmittel kommen bei der Variante α des erfindungsgemässen Verfahrens (e) alle üblicherweise für derartige Umsetzungen gebräuchlichen Reagenzien in Frage. Hierzu gehören anorganische und organische Basen, wie Alkalimetall- und Erdalkalimetallhydroxide bzw. -carbonate und hydrogencarbonate, ferner organische Amine, wie tertiäre aliphatische Amine, z.B. Triethylamin. Besonders bevorzugt sind nicht quaternierbare aliphatische Amine, wie Ethyldiisopropylamin (Hünig-Base).

Als Verdünnungsmittel kommen bei der Variante α des erfindungsgemässen Verfahrens (e) alle inerten organischen Solventien in Betracht. Vorzugsweise verwendbar sind Äther, wie Diethyläther, Dioxan oder Tetrahydrofuran. Es ist ferner möglich, ein zur Halogenwasserstoffabspaltung verwendetes Amin gleichzeitig als Lösungsmittel zu benutzen. Ausserdem kann auch Wasser als Verdünnungsmittel fungieren.

Die Reaktionstemperaturen können bei der Variante α des erfindungsgemässen Verfahrens (e) innerhalb eines grösseren Bereiches variiert werden. Im allgemeinen arbeitet man bei Temperaturen zwischen 0 und 100°C, vorzugsweise zwischen 10 und 50°C.

Bei der Durchführung der Variante α des erfindungsgemässen Verfahrens (e) setzt man auf 1 mol eines 4,5-Dichlorimidazolderivates der Formel VI 2 mol oder einen grösseren nahezu beliebigen Überschuss an Dehydrohalogenierungsmittel. Die Isolierung des Reaktionsproduktes erfolgt nach üblichen Methoden. Im allgemeinen geht man so vor, dass man das Reaktionsgemisch in

Wasser giesst, ansäuert und das kristallin anfallende Reaktionsprodukt abfiltriert.

Bei der Durchführung der Variante β des erfindungsgemässen Verfahrens (e) wird ein 4,5-Dichlorimidazolderivat der Formel VI mit Wasser hydrolysiert. Das Wasser fungiert hierbei gleichzeitig als Reaktionsmedium und wird deshalb in grossem Überschuss eingesetzt.

Als säurebindende Mittel können bei der Variante β des erfindungsgemässen Verfahrens (e) alle üblichen Basen oder basischen Salze verwendet werden. Vorzugsweise geeignet sind Alkalimetallsalze niederer aliphatischer Carbonsäuren, wobei Natriumformiat speziell genannt sei.

Die Reaktionstemperaturen können bei der Variante β des erfindungsgemässen Verfahrens (e) innerhalb eines grösseren Bereiches variiert werden. Im allgemeinen arbeitet man bei Temperaturen zwischen 0 und 150°C, vorzugsweise zwischen 10 und 120°C.

Bei der Durchführung der Variante β des erfindungsgemässen Verfahrens (e) setzt man auf 1 mol an 4,5-Dichlorimidazolderivat der Formel VI einen grossen Überschuss an Wasser ein, wobei zur Erzielung eines vollständigen Umsatzes die bei der Hydrolyse entstehende Halogenwasserstoffsäure laufend aus dem Reaktionsgemisch entfernt werden muss. Das kann prinzipiell dadurch geschehen, dass man durch kontrollierte Zugabe von insgesamt einem Mol einer beliebigen üblichen Base den pH-Wert vom stark sauren (pH 1-2) in den schwach sauren bis neutralen Bereich (pH 5-7) bringt, mit der Massgabe, dass zur Vermeidung von Nebenreaktionen der pH-Wert 7 keinesfalls überschritten werden darf. In einer bevorzugten Ausführungsform der Variante β des erfindungsgemässen Verfahrens (e) führt man die Hydrolyse in Gegenwart von mindestens 1 mol eines Alkalimetallsalzes einer niederen aliphatischen Carbonsäure durch. Bevorzugt verwendbar ist hierbei Natriumformiat, das in einem beliebigen Überschuss eingesetzt werden kann. Die Isolierung des Reaktionsproduktes erfolgt nach üblichen Methoden. Im allgemeinen verfährt man in der Weise, dass man die wässrige Phase in der Hitze abtrennt, erkalten lässt und das sich dabei kristallin abscheidende Reaktionsprodukt abfiltriert.

Die bei dem erfindungsgemässen Verfahren (f) als Ausgangssubstanzen benötigten 4,5-Dichlorimidazolderivate sind durch die Formel VII definiert.

Die Verbindungen der Formel VII sind bisher noch nicht bekannt. Sie lassen sich jedoch nach dem erfindungsgemässen Verfahren (b), Variante α, bzw. nach dem erfindungsgemässen Verfahren (e), Variante β, herstellen.

Die bei dem erfindungsgemässen Verfahren (f) weiterhin als Ausgangsstoffe benötigten Verbindungen sind durch die Formel VIII definiert.

Die Verbindungen der Formel VIII sind bekannt oder lassen sich nach im Prinzip bekannten Verfahren herstellen.

Als Verdünnungsmittel können bei dem erfindungsgemässen Verfahren (f) alle üblichen inerten organischen Lösungsmittel sowie Wasser verwendet werden.

Die Reaktionstemperaturen können bei dem erfindungsgemässen Verfahren (f) innerhalb eines grösseren Bereiches variiert werden. Im allgemeinen arbeitet man bei Temperaturen zwischen 20 und 120°C, vorzugsweise zwischen 80 und 110°C.

Bei der Durchführung des erfindungsgemässen Verfahrens (f) setzt man auf 1 mol an 4,5-Dichlorimidazolderivat der Formel VII mindestens 1 mol einer Verbindung der Formel VIII ein. Die Isolierung der Reaktionsprodukte erfolgt nach üblichen Methoden. Im allgemeinen verfährt man in der Weise, dass man bei Verwendung von Wasser als Verdünnungsmittel den gegebenenfalls nach vorherigem Ansäuern anfallenden Niederschlag abfiltriert. Dient ein organisches Solvens als Lösungsmittel, so engt man nach beendeter Umsetzung ein, versetzt mit Wasser, erhitzt und verfährt dann in der vorstehend beschriebenen Weise.

Das bei dem erfindungsgemässen Verfahren (g) als Ausgangsprodukt benötigte 4,5-Dichlor-2-ethinylimidazol ist durch die Formel IX definiert. Der Stoff ist bisher noch nicht bekannt. Er lässt sich jedoch nach dem erfindungsgemässen Verfahren (e), Variante α, herstellen.

Als Chlorierungs- bzw. Bromierungsmittel können bei dem erfindungsgemässen Verfahren (g) alle üblichen für derartige Zwecke verwendbaren Reagenzien eingesetzt werden. Bevorzugt in Frage kommen Chlor, Sulfurylchlorid und Brom.

Als Verdünnungsmittel können bei dem erfindungsgemässen Verfahren (g) alle üblichen inerten organischen Solventien eingesetzt werden. Vorzugsweise in Betracht kommen Chloroform, Methylenchlorid und Tetrachlorkohlenstoff.

Die Reaktionstemperaturen können bei dem erfindungsgemässen Verfahren (g) innerhalb eines bestimmten Bereiches variiert werden. Im allgeimeinen arbeitet man bei Temperaturen zwischen 0 und 50°C, vorzugsweise zwischen 10 und 30°C.

Bei der Durchführung des erfindungsgemässen Verfahrens (g) setzt man das 4,5-Dichlor-2-ethinylimidazol der Formel IX je nach dem gewünschten Endprodukt mit unterschiedlichen Mengen an Chlorierungs- bzw. Bromierungsmittel um. Soll ein Endprodukt hergestellt werden, in dem die Seitenkette gesättigt ist, so setzt man auf 1 mol an 4,5-Dichlor-2-ethinylimidazol der Formel IX mindestens 2 mol an Chlorierungs- bzw. Bromierungsmittel ein. Verwendet man nur 1 mol an Chlorierungs- bzw. Bromierungsmittel, so bilden sich Verbindungen mit ungesättigter Seitenkette. Die Isolierung der Reaktionsprodukte erfolgt nach üblichen Methoden. In manchen Fällen wird das Reaktionsprodukt direkt in kristalliner Form erhalten, so dass es abfiltriert werden kann.

Die bei dem erfindungsgemässen Verfahren (h) als Ausgangsstoffe benötigten 4,5-Dichlorimidazolderivate sind durch die Formel X definiert.

Die 4,5-Dichlorimidazolderivate der Formel X sind bisher noch nicht bekannt. Sie lassen sich jedoch nach erfindungsgemässen Verfahren herstellen.

Als Chlorierungs- bzw. Bromierungsmittel können bei dem erfindungsgemässen Verfahren (h) alle üblichen für derartige Zwecke verwendbaren Reagenzien eingesetzt werden. Bevorzugt in Frage kommen Chlor, Sulfurylchlorid und Brom.

Als Verdünnungsmittel können bei dem erfindungsgemässen Verfahren (h) alle üblichen inerten organischen Solventien eingesetzt werden. Vorzugsweise in Betracht kommen Chloroform, Methylenchlorid und Tetrachlorkohlenstoff.

Die Reaktionstemperaturen können bei dem erfindungsgemässen Verfahren (h) innerhalb eines bestimmten Bereiches variiert werden. Im allgemeinen arbeitet man bei Temperaturen zwischen 0 und 50°C, vorzugsweise zwischen 10 und 30°C.

Bei der Durchführung des erfindungsgemässen Verfahrens (h) setzt man auf 1 mol an 4,5-Dichlorimidazolderivat der Formel X 1 mol oder auch einen Überschuss an Chlorierungs- bzw. Bromierungsmittel ein. Die Aufarbeitung erfolgt nach üblichen Methoden. Im allgemeinen verfährt man in der Weise, dass man gegebenenfalls nach vorherigem Einengen das in kristalliner Form anfallende Reaktionsprodukt abfiltriert.

Die bei dem erfindungsgemässen Verfahren (i) als Ausgangsstoffe benötigten 4,5-Dichlorimidazolderivate sind durch die Formel XI definiert.

Die Verbindungen der Formel XI sind bisher noch nicht bekannt. Sie lassen sich jedoch nach erfindungsgemässen Verfahren herstellen.

Als Lösungsmittel kommen bei dem erfindungsgemässen Verfahren (i) alle üblichen niederen aliphatischen Nitrile in Betracht. Vorzugsweise verwendbar ist Acetonitril.

Die Reaktionstemperaturen können bei dem erfindungsgemässen Verfahren (i) innerhalb eines grösseren Bereiches variiert werden. Im allgemeinen arbeitet man bei Temperaturen zwischen 50 und 150°C, vorzugsweise zwischen 60 und 100°C.

Bei der Durchführung des ergindungsgemässen Verfahrens (i) erhitzt man eine Verbindung der Formel XI in einem niederen aliphatischen Nitril einige Zeit. Danach wird nach üblichen Methoden aufgearbeitet. Im allgemeinen verfährt man in der Weise, dass man nach dem Erkalten das Reaktionsgemisch filtriert, dann mit Wasser versetzt und das kristallin anfallende Produkt absaugt.

Die erfindungsgemässen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden.

Dikotyle Unkräuter der Gattungen: *Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.*

Dikotyle Kulturen der Gattungen: *Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.*

Monokotyle Unkräuter der Gattungen: *Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.*

Monokotyle Kulturen der Gattungen: *Oryza, Zea, Triticum, Hordeum, Avena, Secale, Sorghum, Panicum, Saccarum, Ananas, Asparagus, Allium.*

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in ein einjährigen Kulturen eingesetzt werden.

Die erfindungsgemässen Wirkstoffe zeigen neben einer starken Unkrautwirkung insbesondere auch eine sehr gute selektive herbizide Wirksamkeit in verschiedenen Kulturen. So eignen sich die erfindungsgemässen Wirkstoffe zum Beispiel zur selektiven Unkrautbekämpfung in Mais, Baumwolle und Getreide.

Die Wirkstoffe können in die üblichen Formulierungen übergeführt werden, wie Lösungen, Emulsionen, Suspensionen, Pulver, Stäubemittel, Pasten, lösliche Pulver, Granulate, Suspensions-Emulsionskonzentrate, wirkstoffimprägnierte Natur- und synthetische Stoffe und Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische

Kohlenwasserstoffe, wie Cyclohexan oder Paraffine, z.B. Erdölfraktionen, Alkohole, wie Butanol oder Glycol sowie deren Äther und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel, als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischung mit bekannten Herbiziden zur Unkrautbekämpfung Verwendung finden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemässen Wirkstoffe können sowohl vor als auch nach dem Auflaufen der Pflanzen appliziert werden.

Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in grösseren Bereichen schwanken. Sie hängt im wesentlichen von der Art des gewünschten Effekts ab. Im allgemeinen liegen die Aufwandmengen zwischen 0,1 und 10 kg Wirkstoff/ha, vorzugsweise zwischen 0,1 und 5 kg/ha.

Die gute Wirksamkeit der erfindungsgemässen Wirkstoffe geht aus den folgenden Beispielen hervor.

In den folgenden Beispielen wurden die nachstehend formelmässig angegebenen Verbindungen eingesetzt:

A = 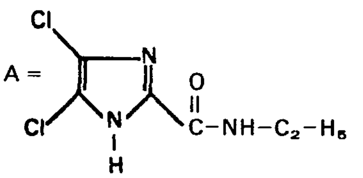 (bekannt)

(4,5-Dichlorimidazol-2-carbonsäure-äthylamid)

(6) = 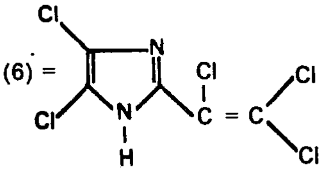

(14) = 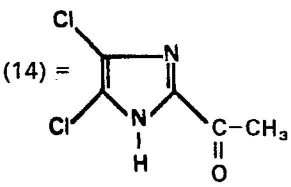

(18) = 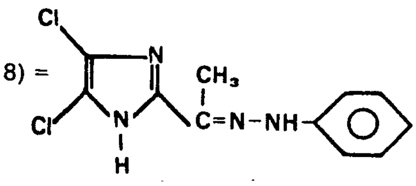

(9) = 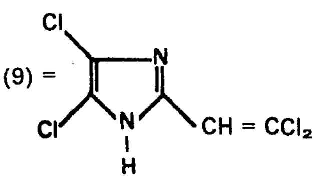

(19) = 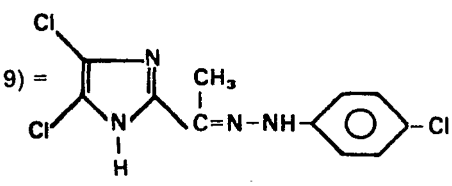

*Beispiel A:*

Pre-emergence-Test

Lösungsmittel: 5 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Tetspflanzen werden in normalen Boden ausgesät und nach 24 h mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach 3 Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Test zeigen die erfindungsgemässen Wirkstoffe (6), (9), (14), (18) und (19) eine bessere Wirksamkeit als die Vergleichssubstanz (A).

*Beispiel B:*

Post-emergence-Test

Lösungsmittel: 5 Gewichtsteile Aceton

Emulgator: 1 Gewichtsteil Alkylarylpolyglykoläther

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 bis 15 cm haben so, dass die jeweils gewünschte Wirkstoffmenge pro Flächeneinheit ausgebracht wird. Die Konzentration der Spritzbrühe wird so gewählt, dass in 2000 l Wasser/ha die jeweils gewünschte Wirkstoffmenge ausgebracht wird. Nach 3 Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

In diesem Test zeigen die erfindungsgemässen Verbindungen (6), (9), (14) und (19) eine bessere Wirksamkeit als die Vergleichssubstanz (A).

*Herstellungsbeispiele*

*Beispiel 1:*

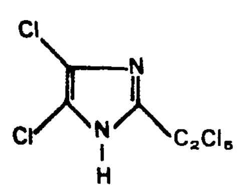

a) In 2 l auf 5°C vorgekühltes Phosphoroxidchlorid wurden unter Rühren und Kühlung 480 g (5 mol) 2-Ethylimidazol eingetragen und anschliessend zwischen 20 und 40°C bis zum Ende der exothermen Reaktion HCl-Gas eingeleitet. Anschliessend wurde auf Rückflusstemperatur (ca. 107°C) erhitzt und dort im Verlauf vom etwa 5 h 2490 g (35 mol) Chlorgas eingeleitet. Nach dem Abdestillieren des Phosphoroxidchlorids im Wasserstrahlvakuum bis zu einer Badtemperatur von 100°C wurde der Rückstand bei Raumtemperatur allmählich mit 1,75 l Ameisensäure verrührt. Das hierbei entstehende farblose, kristalline Pulver wurde abfiltriert, mit Ameisensäure und dann gründlich mit Wasser nachgewaschen. Man erhielt so nach dem Trocknen 520 g (30,8% der Theorie) 4,5-Dichlor-2-pentachlorethylimidazol vom Schmelzpunkt 232°C (lange dünne Nadeln aus Waschbenzin oder weniger wasserfreiem Acetonitril).

IR (KBr): 1540, 1450, 1410, 1358, 1250, 1205, 1040, 780, 665, 555 $cm^{-1}$.

b) 125 g (1,3 mol) 2-Ethylimidazol wurden unter Rühren und Kühlung in 500 ml auf etwa 5°C vorgekühltes $POCl_3$ eingetragen. Anschliessend wurde auf Rückflusstemperatur (ca. 107°C) erhitzt und dort im Verlauf von 3 h 300 g (4,22 mol) Chlor eingeleitet. Das Lösungsmittel wurde im Vakuum abgezogen und der Rückstand bei ca. 20°C mit 400 ml Ameisensäure verrührt. Der hierbei entstehende kristalline Niederschlag wurde wie unter a) isoliert und stellt ebenfalls 4,5-Dichlor-2-pentachlorethylimidazol dar. Ausbeute 63 g, entsprechend 38% der Theorie, bezogen auf das im Unterschuss eingesetzte Chlor.

c) In eine Lösung von 96 g (1,0 mol) 2-Ethylimidazol in 400 ml 1,1,2,2-Tetrachlorethan wurden innerhalb 4,5 h zwischen 110 und 115°C 480 g (6,75 mol) Chlor eingeleitet. Danach wurde das Tetrachlorethan im Wasserstrahlvakuum bis zu einer Badtemperatur von 100°C abgezogen. Der erkaltete Rückstand wurde mit 200 ml reiner Ameisensäure gerührt, worauf sich ein farbloses Pulver abschied, das nach dem Absaugen, Waschen mit Ameisensäure und Trocknen 103 g (31,6% der Theorie) 4,5-Dichlor-2-pentachlorethylimidazol ergab, identisch mit der unter a) erhaltenen Verbindung. Man erhielt die reine Verbindung in einem Wiederholungsansatz auch in der Weise, dass man sie bei etwa 125°C/0,01 Torr aus dem Rohprodukt heraussublimierte.

*Beispiel 2:*

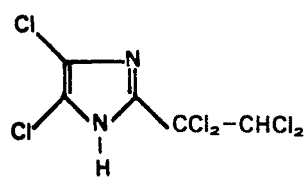

a) Zu einem gerührten Gemisch aus 16,1 g (0,1 mol) 4,5-Dichlor-2-ethinylimidazol (Herstellung siehe Beispiel 13) und 100 ml Chloroform wurden im Verlauf einer Stunde unter Kühlung bei 15 bis 20°C 34 g (0,25 mol) Sulfurylchlorid getropft. Nach zweistündigem Nachrühren wurde der Festkörper abgesaugt und mit wenig Chloroform gewaschen. Man erhielt so 4,5-Dichlor-2-(1,1,2,2-tetrachlorethyl)imidazol vom Schmelzpunkt 180°C.

IR (KBr): 1547, 1465, 1427, 1360, 1260, 1248, 1208, 1055, 1015, 830, 770, 740, 682, 596, 555 $cm^{-1}$.

b) 120 g (1,25 mol) 2-Ethylimidazol wurden unter Rühren und Kühlung in 500 ml Thionylchlorid eingetragen. Anschliessend wurde auf Rückfluss erhitzt (76°C) und bei dieser Temperatur im Verlauf von 5 h 530 g (7,5 mol) Chlor eingeleitet. Nach dem Abdestillieren des Thionylchlorids im Wasserstrahlvakuum bis zu einer Badtemperatur von 100°C wurde der Rückstand bei Raumtemperatur mit etwa 300 ml Ameisensäure verrührt. Das hierbei entstehende, kristalline Pulver wurde abfiltriert, mit Ameisensäure gewaschen und getrocknet. Man erhielt so 72 g (19% der Theorie) 4,5-Dichlor-2-(1,1,2,2-tetrachlorethyl)imidazol, identisch mit der unter a) erhaltenen Verbindung.

*Beispiel 3:*

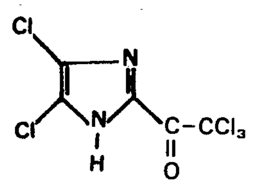

Eine Suspension von 68 g (0,2 mol) 4,5-Dichlor-2-pentachlorethylimidazol in 1,2 l Wasser wurde zum Sieden erhitzt und im Verlauf von 2 h etwa 700 ml Wasser abgedampft. Anschliessend wurde abgesaugt und getrocknet. Ausbeute 46 g (81% der Theorie) 4,5-Dichlor-2-trichloracetylimidazol vom Schmelzpunkt 199°C.

IR (KBr): 1700, 1525, 1410, 1270, 1200, 1090, 1050, 977, 845, 810, 754, 700, 660, 621, 609, 527 $cm^{-1}$.

*Beispiel 4:*

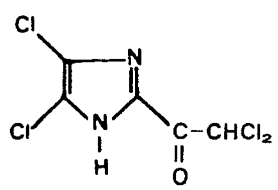

In analoger Weise wie in Beispiel 3 wurde aus 4,5-Dichlor-2-(1,1,2,2-tetrachlorethyl)imidazol das 4,5-Dichlor-2-dichloracetylimidazol vom Schmelzpunkt 102°C gewonnen.

IR (KBr): 1692, 1535, 1423, 1412, 1190, 1075, 965, 808, 785, 758, 608 $cm^{-1}$.

*Beispiel 5:*

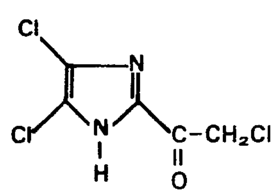

In analoger Weise wie in Beispiel 3 wurde aus 4,5-Dichlor-2-(1,1,2-trichlorethyl)imidazol (Herstellung siehe Beispiel 21) das 4,5-Dichlor-2-chloracetylimidazol vom Schmelzpunkt 182°C (aus wenig Acetonitril) gewonnen.

IR (KBr): 1683, 1415, 1329, 1185, 1062, 1045, 945, 781, 740 $cm^{-1}$.

*Beispiel 6:*

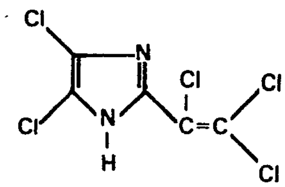

In eine Lösung von 150 g (1 mol) Natriumjodid in 900 ml Aceton wurden bei Rautemperatur unter Rühren 50 g (0,148 mol) 4,5-Dichlor-2-pentachlorethylimidazol eingetragen. Nach einstündigem Weiterrühren wurden etwa 500 ml Aceton abgezogen und der Rückstand mit 2 l Wasser und so viel 40%iger wässriger $NaHSO_3$- Lösung versetzt, bis die braune Jodfarbe völlig verschwunden war. Anschliessend wurde abgesaugt, mit Wasser gewaschen und getrocknet. Ausbeute 37 g (93,5% der Theorie) 4,5-Dichlor-2-trichlorvinylimidazol vom Schmelzpunkt 168°C.

IR (KBr): 1590, 1542, 1458, 1405, 1265, 1061, 1042, 965, 958, 869, 770, 700, 556 $cm^{-1}$.

*Beispiel 7:*

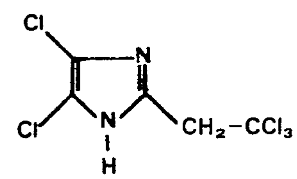

In eine Lösung von 285 g (7,5 mol) Natriumboranat in 1,3 l Wasser wurden unter Rühren und Kühlen bei 15 bis 25°C im Verlauf von etwa 5 h 506 g (1,5 mol) 4,5-Dichlor-2-pentachlorethylimidazol eingetragen. Nach dreistündigem Nachrühren wurde unter Kühlung mit Salzsäure auf etwa pH = 1 angesäuert. Der Niederschlag wurde abgesaugt, anschliessend mit etwa 4 l Wasser unter Rühren aufgekocht, siedend heiss filtriert und mit heissem Wasser mehrmals nachgewaschen (Entfernung der Borsäure). Nach dem Trocknen erhielt man so 377 g (93,5% der Theorie) 4,5-Dichlor-2-(2,2,2-trichlorethyl)imidazol vom Schmelzpunkt 224°C (Zers.).

IR (KBr): 1557, 1425, 1370, 1248, 1222, 1194, 1045, 1035, 1011, 946, 840, 730, 709, 558 $cm^{-1}$.

*Beispiel 8:*

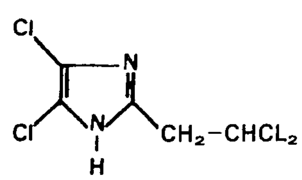

In analoger Weise wie in Beispiel 7 wurde aus 4,5-Dichlor-2-(1,1,2,2-tetrachlorethyl)imidazol das 4,5-Dichlor-2-(2,2-dichlorethyl)imidazol vom Schmelzpunkt 143°C (aus Cyclohexan) gewonnen.

IR (KBr): 1577, 1560, 1435, 1410, 1215, 1038, 1015, 925, 810, 720, 688, 648 $cm^{-1}$.

*Beispiel 9:*

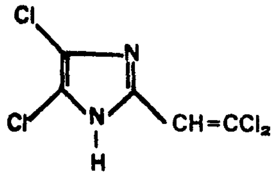

a) 4,5-Dichlor-2-(2,2,2-trichlorethyl)imidazol wurde in überschüssige gesättigte, wässrige Natriumhydrogencarbonatlösung eingetragen und bis zum Sieden erhitzt, wobei eine klare Lösung entstand. Nach dem Abkühlen wurde mit verdünnter Salzsäure angesäuert, der ausgefallene Niederschlag abgesaugt, mit Wasser gewaschen und getrocknet. Man erhielt so 4,5-Dichlor-2-(2,2-dichlorvinyl)imidazol vom Schmelzpunkt 134°C. Die Verbindung kann bei 110°C/0,01 Torr sublimiert werden.

IR (KBr): 1625, 1550, 1465, 1414, 1235, 1035, 920, 835, 675, 539 $cm^{-1}$.

b) Eine Mischung aus 67 g (0,25 mol) 4,5-Dichlor-2-(2,2,2-trichlorethyl)imidazol, 200 ml Dioxan und 28 g (0,277 mol) Triethylamin wurde 1 h unter Rückfluss gerührt. Nach dem Erkalten wurde in 1 l Wasser gegossen, mit Salzsäure angesäuert, abgesaugt, mit Wasser gewaschen und getrocknet. Die nachfolgende Sublimation bei 110°C/0,01 Torr lieferte 47,5 g (82%) 4,5-Dichlor-2-(2,2-dichlorvinyl)imidazol, identisch mit der unter a) erhaltenen Verbindung.

*Beispiel 10:*

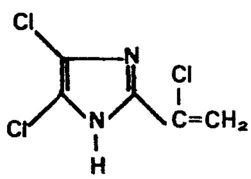

a) In einem Glasautoklav wurde eine Lösung von 50 g (0,186 mol) 2,4,5-Trichlor-2-(1,1-dichlorethyl)-2H-imidazol in 450 ml Toluol mit 5 g Katalysator (5% Palladium auf Kohle) versetzt und bei etwa 20°C im Verlauf von 5 h hydriert. Das Reaktionsgemisch wurde im Vakuum zur Trockne einrotiert, der Rückstand in Wasser suspendiert und mit wässriger Natriumhydrogencarbonatlösung neutralisiert. Anschliessend wurde abgesaugt, mit Wasser gewaschen und getrocknet. Nachfolgende Extraktion mit heissem Acetonitril lieferte nach dem Erkalten und gegebenenfalls Einengen der filtrierten Acetonitrillösung 4,5-Dichlor-2-(1-chlorvinyl)imidazol als derbe, farblose Kristalle vom Schmelzpunkt 210°C (Zers.). Die Verbindung kann bei 100°C/0,1 Torr sublimiert werden.

b) Eine Mischung aus 67 g (0,25 mol) 2,4,5-Trichlor-2-(1,1-dichlorethyl)-2H-imidazol, 28,3 g (0,275 mol) Natriumbromid und 350 ml Aceton wurde 1 h unter Rückfluss (58°C) gerührt. Nach dem Erkalten wurde in 2 l Wasser gegossen, der Niederschlag abgesaugt, mit Wasser gewaschen und getrocknet. Man erhielt 41 g (83% der Theorie) 4,5-Dichlor-2-(1-chlorvinyl)imidazol, identisch mit der unter a) erhaltenen Verbindung.

IR (KBr): 1622, 1550, 1477, 1430, 1355, 1265, 1120, 1050, 1009, 883, 795, 734 $cm^{-1}$.

*Beispiel 11:*

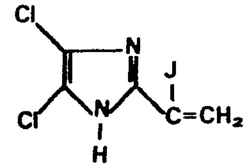

In eine gerührte Mischung aus 1600 g (10,6 mol) Natriumjodid in 2 l Aceton wurde unter Kühlung im Verlauf von 2 h bei maximal 50°C eine Lösung von 806 g (3 mol) 2,4,5-Trichlor-2-(1,1-dichlorethyl)-2H-imidazol (Herstellung siehe DE-OS Nr. 2550157) in 1,5 l Aceton getropft. Anschliessend wurde in eine Mischung aus 12 l Wasser und 1600 g 40%iger wässrige $NaHSO_3$-Lösung eingetragen, abgesaugt, mit Wasser gewaschen und getrocknet. Ausbeute 770 g (89% der Theorie) 4,5-Dichlor-2-(1-jodvinyl)imidazol. Zersetzungspunkt etwa 215°C.

*Beispiel 12:*

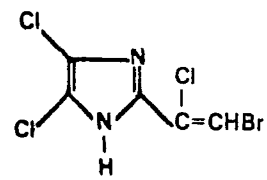

a) Eine Mischung aus 400 g (3,36 mol) Kaliumbromid, 113 g (0,5 mol) 2,4,5-Trichlor-2-(1,1-dichlorethyl)-2H-imidazol und 1,5 l Acetonitril wurde 10 h unter Rückfluss (81°C) gerührt. Nach dem Erkalten wurde auf 7,5 l Wasser gegeben, der Niederschlag abgesaugt, mit Wasser gewaschen und getrocknet. Man erhielt 121 g (87% der Theorie) 4,5-Dichlor-2-(1-chlor-2-bromvinyl)imidazol. Schmelzpunkt 190°C (aus Toluol).

IR (KBr): 1585, 1540, 1465, 1402, 1370, 1260, 1205, 1050, 895, 870, 775, 740, 690, 622, 540 $cm^{-1}$.

b) 3,58 g (0,01 mol) der Verbindung der Formel

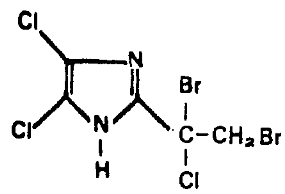

(Herstellung siehe Beispiel 22)

wurden in 30 ml Acetonitril 5 min gekocht. Nach dem Erkalten wurde auf 200 ml Wasser gegeben, der Niederschlag abgesaugt, mit Wasser gewaschen und getrocknet. Man erhielt 2,60 g (94% der

Theorie) 4,5-Dichlor-2-(1-chlor-2-bromvinyl)-imidazol, identisch mit der unter a) erhaltenen Verbindung.

*Beispiel 13:*

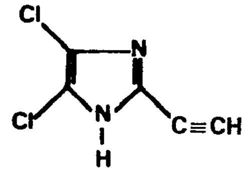

a) Eine Suspension von 28,9 g (0,1 mol) 4,5-Dichlor-2-(1-jodvinyl)imidazol in 50 ml Dioxan wurde bei Raumtemperatur mit 25 g (0,25 mol) Triethylamin versetzt, wobei unter Erwärmen eine Lösung entstand. Nach halbstündigem Nachrühren wurde in 1 l Wasser gegossen, mit verdünnter Salzsäure schwach angesäuert, abgesaugt, mit Wasser gewaschen und getrocknet. Ausbeute 11,1 g (69% der Theorie) 4,5-Dichlor-2-ethyinylimidazol. Zersetzungspunkt etwa 185°C. Die Verbindung ist bei 100°C/0,01 Torr sublimierbar.

b) Eine Lösung von 28,9 g (0,1 mol) 4,5-Dichlor-2-(1-jodvinyl)imidazol in 150 ml Dioxan wurde bei 20°C mit 32,3 g (0,25 mol) Ethyldiisopropylamin (Hünig-Base) versetzt. Nach einstündigem Nachrühren wurde in 750 ml Wasser gegossen und mit verdünnter Salzsäure schwach angesäuert, abgesaugt, mit Wasser gewaschen und getrocknet. Ausbeute 11,7 g 4,5-Dichlor-2-ethinylimidazol. Das Filtrat wurde im Vakuum auf etwa ein Drittel eingeengt, wonach weitere 4,3 g 4,5-Dichloro-2-ethinylimidazol isoliert werden konnten.

Gesamtausbeute 16,0 g (99% der Theorie).

IR (KBr): 3295, 2125, 1563, 1545, 1470, 1390, 1050, 1020, 875, 702, 688, 610 $cm^{-1}$.

*Beispiel 14:*

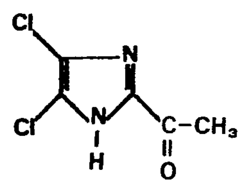

a) Zu einer unter Rückfluss siedenden, gerührten Lösung von 136 g (2 mol) Natriumformiat in 4 l Wasser wurden portionsweise 289 g (1 mol) 4,5-Dichlor-2-(1-jodvinyl)imidazol gegeben und bis zu dessen Auflösung weiter unter Rückfluss gerührt. Danach trennt man noch siedend heiss von etwas dunklem Öl am Boden des Reaktionskolbens ab und lässt die klare wässrige Lösung erkalten. Es scheiden sich zentimeterlange, hellgelbe Nadeln ab, die kalt abgesaugt, mit wenig kaltem Wasser gewaschen und getrocknet werden. Ausbeute 125 g (70% der Theorie) 2-Acetyl-4,5-dichlorimidazol vom Schmelzpunkt 162°C.

b) Ein Wiederholungsansatz, bei dem statt reinem Wasser die Mutterlauge des vorstehend beschriebenen Ansatzes verwendet wurde, lieferte etwa 80% Ausbeute an 2-Acetyl-4,5-dichlorimidazol.

c) Arbeitete man analog a) mit dem Unterschied, dass statt 4,5-Dichlor-2-(1-jodvinyl)-imidazol die entsprechend molare Menge 4,5-Dichlor-2-(1-chlorvinyl)imidazol eingesetzt wurde, so erhielt man in vergleichbaren Ausbeuten ebenfalls 2-Acetyl-4,5-dichlorimidazol.

IR (KBr): 1666, 1432, 1408, 1212, 1050, 993, 950, 785, 693, 619, 580, 552, 512, 492 $cm^{-1}$.

*Beispiel 15:*

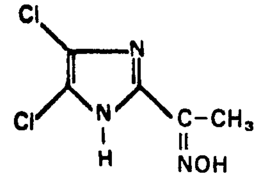

Eine Mischung aus 8,95 g (0,05 mol) 2-Acetyl-4,5-dichlorimidazol, 200 ml Wasser und 6,95 g (0,1 mol) Hydroxylaminhydrochlorid wurde zunächst bei Raumtemperatur anteilweise mit 4,60 g (0,055 mol) Natriumhydrogencarbonat versetzt und anschliessend zum Sieden erhitzt, worauf eine klare Lösung entstand. Der beim Erkalten auskristallisierende Niederschlag wurde abgesaugt, mit Wasser gewaschen und getrocknet. Ausbeute 8,7 g (97% der Theorie) Oxim der obigen Strukturformel vom Schmelzpunkt 213°C (Zers.).

*Beispiel 16:*

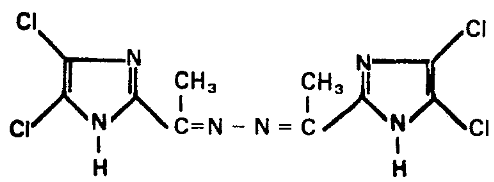

8,95 g (0,1 mol) 2-Acetyl-4,5-dichlorimidazol wurden in einer Mischung aus 20 g Hydrazinhydrat und 200 ml Wasser gelöst und kurz zum Sieden erhitzt. Nach dem Abkühlen wurde mit Salzsäure angesäuert, worauf ein Niederschlag ausfiel. Nach dem Absaugen, Waschen mit Wasser und Trocknen erhielt man in praktisch quantitativer Ausbeute das Azin der obigen Strukturformel. Der Zersetzungspunkt liegt oberhalb 290°C.

*Beispiel 17:*

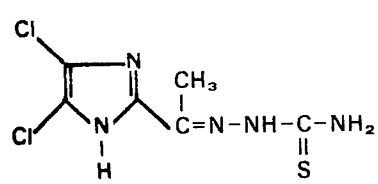

17,9 g (0,1 mol) 2-Acetyl-4,5-dichlorimidazol wurden in 800 ml Wasser in der Hitze gelöst und mit einer heissen Lösung von 15 g (0,165 mol) Thiosemicarbazid in 200 ml Wasser versetzt. Nach etwa einer halben Minute entstand ein farbloser Niederschlag, der nach dem Abkühlen abgesaugt, mit Wasser gewaschen und getrocknet wurde. Man erhielt so das Thiosemicarbazin der obigen

Strukturformel vom Schmelzpunkt oberhalb 290°C.

*Beispiel 18:*

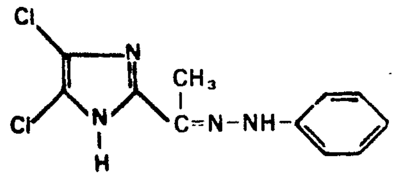

17,9 g (0,1 mol) 2-Acetyl-4,5-dichlorimidazol wurden in 800 ml Wasser in der Hitze gelöst, mit 14 g (0,13 mol) Phenylhydrazin versetzt und kurz zum Sieden erhitzt. Nach dem Erkalten wurde abgesaugt, mit Wasser gewaschen und getrocknet. Man erhielt in praktisch quantitativer Ausbeute das Phenylhydrazon der obigen Strukturformel vom Schmelzpunkt 175°C.

*Beispiel 19:*

In analoger Weise wurde das 4-Chlorphenylhydrazon der Strukturformel

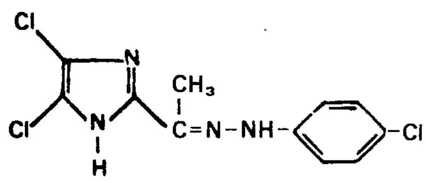

gewonnen. Schmelzpunkt 240°C (Zers.)

*Beispiel 20:*

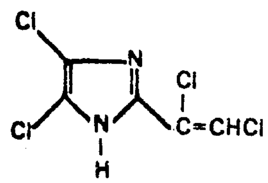

a) 13,4 g (0,05 mol) der Verbindung der Formel

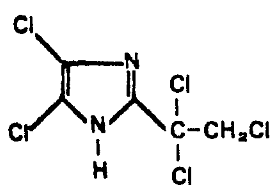

(Herstellung siehe Beispiel 21)
wurden in 50 ml Acetonitril 10 min gekocht. Der nach dem Erkalten auskristallisierte Niederschlag wurde abgesaugt, mit etwas kaltem Acetonitril gewaschen und getrocknet. Anschliessend wurde er bei etwa 20°C mit Wasser verrührt, abgesaugt, mit Wasser gewaschen und getrocknet. Man erhielt so 4,5-Dichlor-2-(1,2-dichlorvinyl)imidazol vom Schmelzpunkt 198°C (aus wenig Toluol).

IR (KBr): 1591, 1545, 1468, 1407, 1375, 1257, 1055, 916, 694, 632, 548 $cm^{-1}$.

b) Eine Lösung von 50 g (0,33 mol) Natriumjodid in 150 ml Aceton wurde mit 26,8 g (0,0772 mol) der Verbindung der Formel

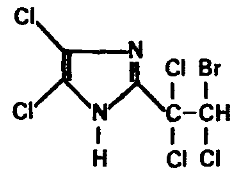

(Herstellung siehe Beispiel 23) versetzt und anschliessend 2 h bei Raumtemperatur nachgerührt. Danach wurde in etwa 1,5 l Wasser gegeben und das freie Jod durch Zugabe von 40%iger wässriger $NaHSO_3$-Lösung bis zur Entfärbung entfernt. Durch nachfolgendes Absaugen, Waschen mit Wasser und Trocknen erhielt man 17,1 g (95,5% der Theorie) 4,5-Dichlor-2-(1,2-dichlorvinyl)-imidazol, identisch mit der unter a) erhaltenen Verbindung.

c) Zu einer Lösung von 8,05 g (0,05 mol) der Verbindung der Formel

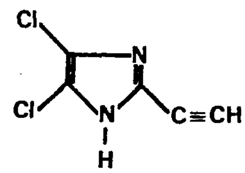

(Herstellung siehe Beispiel 13)
in 50 ml Chloroform wurde innerhalb einer Stunde bei 10 bis 15°C eine Mischung aus 6,75 g (0,05 mol) Sulfurylchlorid und 50 ml Chloroform getropft. Anschliessend wurde 2 h nachgerührt. Danach wurde das Chloroform im Vakuum abgezogen, der Rückstand mit ca. 100 ml Wasser bei 20°C verrührt, abgesaugt, mit Wasser nachgewaschen und getrocknet. Das Rohprodukt wurde zwecks Reinigung anschliessend bei 100°C/0,01 Torr sublimiert. Man erhielt farblose Kristalle von 4,5-Dichlor-2-(1,2-dichlorvinyl)imidazol, identisch mit der unter a) erhaltenen Verbindung.

*Beispiel 21:*

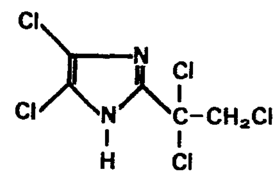

In einem gerührten Gemisch aus 19,75 g (0,1 mol) 4,5-Dichlor-2-(1-chlorvinyl)imidazol und 100 ml reinem Chloroform wurden unter Kühlung bei etwa 10°C im Verlauf von 2 h 15,0 (0,11 mol) Sulfurylchlorid in etwa 40 ml reinem Chloroform getropft.

Anschliessend wurde etwa 3 h bei Raumtemperatur nachgerührt. Danach wurde abgesaugt, mit Chloroform gewaschen und getrocknet. Man erhielt 19,5 g (72,5% der Theorie) farbloses, kristallines 4,5-Dichlor-2-(1,1,2-trichlorethyl)imidazol vom Schmelzpunkt 140°C (Zers.). Weitere Mengen des Produktes können durch Einengen der Mutterlauge gewonnen werden.

IR (KBr): 1545, 1465, 1420, 1360, 1254, 1215, 1185, 1075, 1040, 1010, 950, 812, 760, 735, 688, 590 $cm^{-1}$.

*Beispiel 22:*

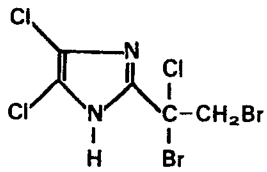

Zu einem gerührten Gemisch aus 19,75 g (0,1 mol) 4,5-Dichlor-2-(1-chlorvinyl)imidazol und 100 ml reinem Chloroform wurden unter Kühlung bei etwa 10°C in 1 h 18,0 g (0,112 mol) Brom in 50 ml reinem Chloroform getropft. Nach vierstündigem Nachrühren bei Raumtemperatur wurde abgesaugt, mit Chloroform gewaschen und getrocknet. Man erhielt 32,0 g (89,5% der Theorie) kristallines 4,5-Dichlor-2-(1-chlor-1,2-dibromethyl)imidazol vom Schmelzpunkt 160°C (Zers.).

IR (KBr): 1542, 1462, 1417, 1357, 1253, 1219, 1168, 1072, 1040, 1004, 918, 795, 760, 680, 647, 618, 575 $cm^{-1}$.

*Beispiel 23:*

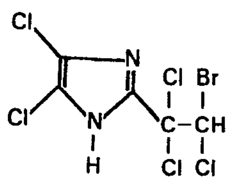

In ähnlicher Weise wie in Beispiel 21 wurde aus 4,5-Dichlor-2-(1-chlor-2-bromvinyl)imidazol das 4,5-Dichlor-2-(1,1,2-trichlor-2-bromethyl)-imidazol vom Schmelzpunkt 180°C (Zers.) (aus Toluol) gewonnen.

IR (KBr): 1545, 1462, 1425, 1357, 1257, 1208, 1170, 1050, 1011, 825, 809, 767, 747, 680, 655 $cm^{-1}$.

*Beispiel 24:*

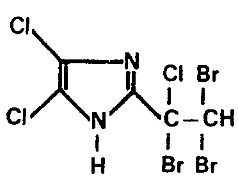

In ähnlicher Weise wie in Beispiel 22 wurde aus 4,5-Dichlor-2-(1-chlor-2-bromvinyl)imidazol das 4,5-Dichlor-2-(1-chlor-1,2,2-tribromethyl)-imidazol in 91,5%iger Ausbeute gewonnen.

IR (KBr): 1540, 1455, 1415, 1350, 1253, 1204, 1034, 1005 $cm^{-1}$.

*Beispiel 25:*

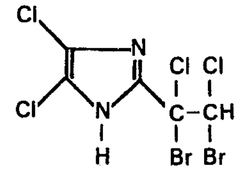

In ähnlicher Weise wie in Beispiel 22 wurde aus 4,5-Dichlor-2-(1,2-dichlorvinyl)imidazol das 4,5-Dichlor-2-(1,2-dichlor-1,2-dibromethyl)-imidazol vom Schmelzpunkt 150°C (Zers.) gewonnen.

IR (KBr): 1540, 1454, 1415, 1350, 1252, 1230, 1203, 1165, 1135, 1043, 1005 $cm^{-1}$.

*Beispiel 26:*

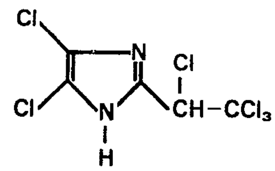

Zu einem gerührten Gemisch aus 5,9 g (0,0255 mol) 4,5-Dichlor-2-(2,2-dichlorvinyl)imidazol und 50 ml reinem Chloroform wurden unter Kühlung bei etwa 10°C in ½ h 6 g (0,044 mol) Sulfurylchlorid in 30 ml Chloroform getropft. Nach zweistündigem Nachrühren wurde die klare Lösung im Vakuum zur Trockne einrotiert. Der Rückstand wurde aus Acetonitril umkristallisiert. Man erhielt farblose Kristalle von 4,5-Dichlor-2-(1,2,2,2-tetrachlorethyl)imidazol vom Schmelzpunkt 230°C (Zers.).

IR (KBr): 1540, 1463, 1449, 1420, 1391, 1305, 1260, 1237, 1037, 782, 759, 699, 684, 625 $cm^{-1}$.

*Beispiel 27:*

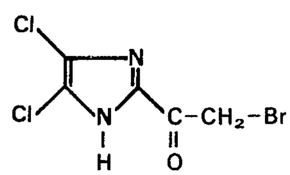

1,79 g (0,005 mol) 4,5-Dichlor-2-(1-chlor-1,2-dibromethyl)imidazol (Herstellung siehe Beispiel 22) wurden in einer Lösung von 3,4 g (0,05 mol) Natriumformiat in 50 ml Wasser 10 min bei Raumtemperatur gerührt. Danach wurde abgesaugt, mit Wasser gewaschen und getrocknet. Man erhielt so 1,20 g (93% der Theorie) 4,5-Dichlor-2-bromacetylimidazol. Nach dem Umkristallisieren aus Cyclohexan schmolz die Verbindung bei 177°C.

IR (KBr): 1676, 1421, 1408, 1372, 1177, 1063, 1049, 949, 767, 669 $cm^{-1}$.

Die in den nachstehenden Beispielen aufgeführten Verbindungen werden nach Verfahren hergestellt, die in den vorausgehenden Beispielen beschrieben sind.

| Beispiel Nr. | Formel | Schmelzpunkt (°C) (umkristallisiert aus) | Ausgangsverbindung siehe Beispiel Nr. | Herstellung analog Beispiel Nr. |
|---|---|---|---|---|
| 28 | 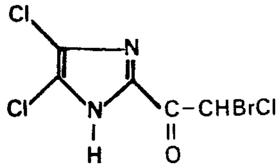 | 128 (Cyclohexan) | 25 | 27 |
| 29 | 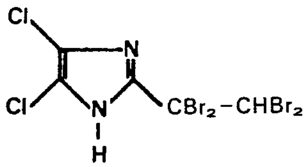 | 150 (Zersetzung) | 13 | 22 |
| 30 | 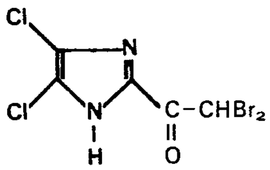 | 140 (Cyclohexan) | 29 | 27 |
| 31 | 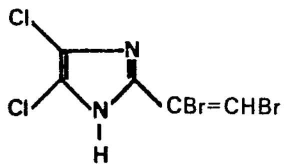 | 191 (Cyclohexan) | 29 | 6 |
| 32 | 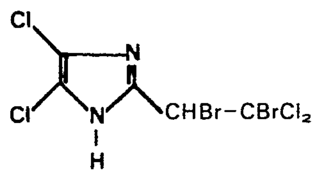 | 220 (Zersetzung) ($CCl_4$) | 9 | 22 |
| 33 | 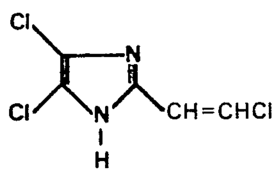 | 223 (Toluol) | 8 | 9 |

## Patentansprüche

1. 4,5-Dichlorimidazolderivate der Formel I

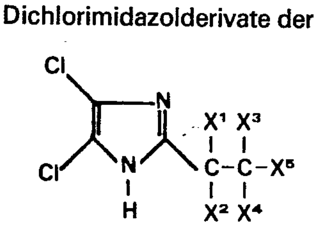

(I)

in welcher

$X^1$ für Wasserstoff, Chlor, Brom oder Jod steht,

$X^2$, $X^3$ $X^4$ jeweils für Wasserstoff, Chlor oder Brom stehen,

$X^5$ für Wasserstoff oder Chlor steht, und ausserdem

$X^1$ und $X^3$ gemeinsam für eine C-C-Bindung stehen können und auch

$X^2$ und $X^4$ gemeinsam für eine C-C-Bindung stehen können, oder

$X^1$ und $X^2$ gemeinsam für Sauerstoff oder die Gruppierung = N−Y stehen, in welcher

Y für Hydroxy und die Reste 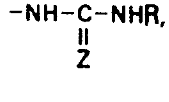

−NH−R¹ oder 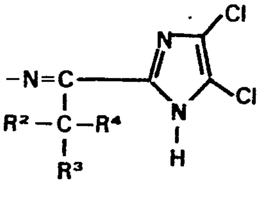 steht,

in welchen

R für Wasserstoff oder Alkyl mit 1 bis 4 Kohlenstoffatomen steht,

$R^1$ für gegebenenfalls durch Fluor, Chlor, Brom, Alkyl mit 1 oder 2 Kohlenstoffatomen, Alkoxy mit 1 oder 2 Kohlenstoffatomen, Trichlormethyl, Trifluormethyl und/oder Nitro substituiertes Phenyl oder Naphthyl steht,

Z für Sauerstoff oder Schwefel steht und

$R^2$, $R^3$ und $R^4$ unabhängig voneinander für Wasserstoff oder Chlor stehen,

wobei jedoch $X^1$, $X^2$, $X^3$, $X^4$ und $X^5$ nicht gleichzeitig für Wasserstoff stehen.

2. Verfahren zur Herstellung von 4,5-Dichlorimidazolderivaten der Formel I

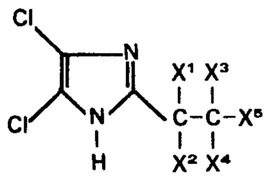

(I)

in welcher

$X^1$ für Wasserstoff, Chlor, Brom oder Jod steht,

$X^2$, $X^3$ und $X^4$ jeweils für Wasserstoff, Chlor oder Brom stehen,

$X^5$ für Wasserstoff oder Chlor steht, und ausserdem

$X^1$ und $X^3$ gemeinsam für eine C-C-Bindung stehen können und auch

$X^2$ und $X^4$ gemeinsam für eine C-C-Bindung stehen können, oder

$X^1$ und $X^2$ gemeinsam für Sauerstoff oder die Gruppierung $=N-Y$ stehen, in welcher

Y für Hydroxy und die Reste 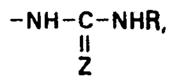

$-NH-R^1$ oder 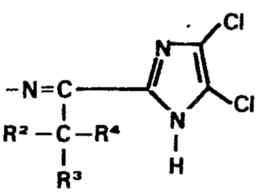 steht,

in welchen

R für Wasserstoff oder Alkyl mit 1 bis 4 Kohlenstoffatomen steht,

$R^1$ für gegebenenfalls durch Fluor, Chlor, Brom, Alkyl mit 1 oder 2 Kohlenstoffatomen, Alkoxy mit 1 oder 2 Kohlenstoffatomen, Trichlormethyl, Trifluormethyl und/oder Nitro substituiertes Phenyl oder Naphthyl steht,

Z für Sauerstoff oder Schwefel steht und

$R^2$, $R^3$ und $R^4$ unabhängig voneinander für Wasserstoff oder Chlor stehen,

wobei jedoch $X^1$, $X^2$, $X^3$, $X^4$ und $X^5$ nicht gleichzeitig für Wasserstoff stehen,

dadurch gekennzeichnet, dass man

a) 2-Ethylimidazol der Formel II

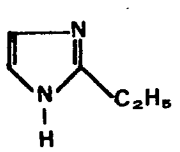

(II)

oder dessen Hydrochlorid mit bis zu 7 mol Chlor/mol an 2-Ethylimidazol in einem unter den Reaktionsbedingungen inerten Lösungsmittel umsetzt,

oder

b) ein 4,5-Dichlorimidazolderivat der Formel III

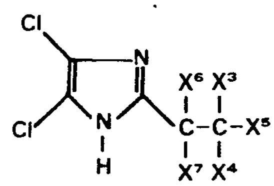

(III)

in welcher

$X^6$ für Chlor oder Brom steht,

$X^7$ für Chlor oder Brom steht und

$X^3$, $X^4$ und $X^5$ die oben angegebene Bedeutung haben, wobei jedoch $X^3$, $X^4$ und $X^5$ nicht gleichzeitig für Wasserstoff stehen

α) mit Wasser hydrolysiert,

oder

β) mit mindestens der stöchiometrisch erforderlichen Menge eines Dehalogenierungsmittels gegebenenfalls in Gegenwart eines Verdünnungsmittels umsetzt,

oder

γ) mit mindestens der stöchiometrisch erforderlichen Menge eines zur Hydrierung gebräuchlichen Metallhydrids in Gegenwart eines Verdünnungsmittels umsetzt,

oder

c) ein 4,5-Dichlorimidazolderivat der Formel IV

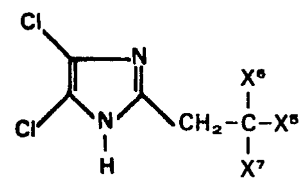

(IV)

in welcher

$X^5$, $X^6$ und $X^7$ die oben angegebene Bedeutung haben, mit einem Chlorwasserstoff abspaltenden Mittel in Gegenwart eines Verdünnungsmittels umsetzt,

oder

d) das 2,4,5-Trichlor-2-(1,1-dichlorethyl)-2H-imidazol der Formel V

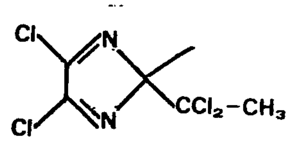

(V)

α) mit einem Reduktionsmittel in Gegenwart eines Verdünnungsmittels umsetzt,

oder

β) mit einem mindestens 3-molaren Überschuss eines Alkalimetalljodids oder mit einem mindestens 1,5-molaren Überschuss eines Erdalkalimetalljodids in Gegenwart eines niederen aliphatischen Ketons als Verdünnungsmittel umsetzt,

oder

γ) mit einer mindestens äquimolaren Menge eines Alkalimetallbromids oder mit einer mindestens

äquivalenten Menge eines Erdalkalimetallbromids in Gegenwart eines niederen aliphatischen Nitrils oder eines niederen aliphatischen Ketons als Verdünnungsmittels umsetzt,
oder
e) ein 4,5-Dichlorimidazolderivat der Formel VI

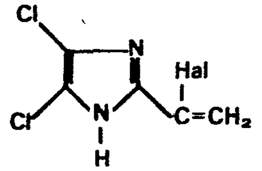

(VI)

in welcher
Hal für Chlor oder Jod steht,
α) mit einem Halogenwasserstoff abspaltenden Mittel gegebenenfalls in Gegenwart eines Verdünnungsmittels umsetzt,
oder
β) mit Wasser in Gegenwart einer Base oder eines basischen Salzes hydrolysiert,
oder
f) ein 4,5-Dichlorimidazolderivat der Formel VII

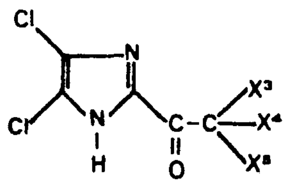

(VII)

in welcher
$X^3$, $X^4$ und $X^5$ die oben angegebene Bedeutung haben,
mit einer Verbindung der Formel VIII

$$H_2N-Y' \quad \text{(VIII)}$$

in welcher
Y' für Amino, Hydroxy und die Reste

$-NH-\underset{\underset{Z}{\|}}{C}-NHR$ oder $-NHR^1$ steht,

in welcher
Z für Sauerstoff oder Schwefel steht,
R für Wasserstoff oder Alkyl mit 1 bis 4 Kohlenstoffatomen steht und
$R^1$ für gegebenenfalls durch Fluor, Chlor, Brom, Alkyl mit 1 oder 2 Kohlenstoffatomen, Alkoxy mit 1 oder 2 Kohlenstoffatomen, Trichlormethyl, Trifluormethyl und/oder Nitro substituiertes Phenyl oder Naphthyl steht, in Gegenwart eines Verdünnungsmittels umsetzt, oder
g) das 4,5-Dichlor-2-ethinylimidazol der Formel IX

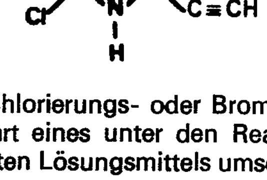

(IX)

mit einem Chlorierungs- oder Bromierungsmittel in Gegenwart eines unter den Reaktionsbedingungen inerten Lösungsmittels umsetzt,
oder
h) 4,5-Dichlorimidazolderivate der Formel X

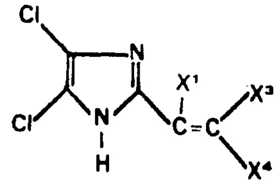

(X)

in welcher
$X^1$, $X^3$ und $X^4$ die oben angegebene Bedeutung haben,
mit einem Chlorierungs- oder Bromierungsmittel in Gegenwart eines unter den Reaktionsbedingungen inerten Lösungsmittels umsetzt,
oder
i) 4,5-Dichlorimidazolderivate der Formel XI

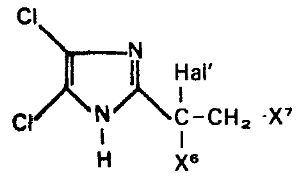

XI)

in welcher
$X^6$ und $X^7$ die oben angegebene Bedeutung haben und
Hal' für Chlor, Brom oder Jod steht,
in Gegenwart eines niederen aliphatischen Nitrils thermisch dehydrohalogeniert.

3. Herbizide Mittel, gekennzeichnet durch einen Gehalt an mindestens einem 4,5-Dichlorimidazolderivat der Formel I gemäss Anspruch 1.

4. Verwendung von 4,5-Dichlorimidazolderivaten der Formel I gemäss Anspruch 1 zur Bekämpfung von Unkräutern.

5. Verfahren zur Herstellung von herbiziden Mitteln, dadurch gekennzeichnet, dass man 4,5-Dichlorimidazolderivate der Formel I gemäss Anspruch 1 mit Streckmitteln und/oder oberflächenaktiven Stoffen vermischt.

## Claims

1. 4,5-Dichloroimidazole derivatives of the formula I

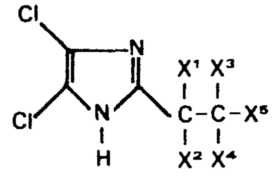

(I)

in which
$X^1$ represents hydrogen, chlorine, bromine or iodine,
$X^2$, $X^3$ and $X^4$ in each case represent hydrogen, chlorine or bromine and
$X^5$ represents hydrogen or chlorine, and in addition,
$X^1$ and $X^3$ together can represent a C-C bond, and also
$X^2$ and $X^4$ together can represent a C-C bond, or

$X^1$ and $X^2$ together represent oxygen or the grouping =N−Y,
in which
Y represents hydroxyl and the radicals

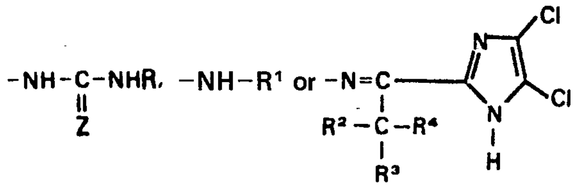

in which:
R represents hydrogen or alkyl with 1 to 4 carbon atoms,
$R^1$ represents phenyl or naphthyl optionally substituted by fluorine, chlorine, bromine, alkyl with 1 or 2 carbon atoms, alkoxy with 1 or 2 carbon atoms, trichloromethyl, trifluoromethyl and/or nitro,
Z represents oxygen or sulphur and
$R^2$, $R^3$ and $R^4$ independently of one another represent hydrogen or chlorine,
but wherein $X^1$, $X^2$, $X^3$, $X^4$ and $X^5$ do not simultaneously represent hydrogen.

2. Process for the preparation of 4,5-dichloroimidazole derivatives of the formula I

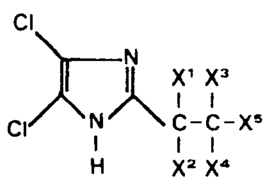
(I)

in which
$X^1$ represents hydrogen, chlorine, bromine or iodine,
$X^2$, $X^3$ and $X^4$ in each case represent hydrogen, chlorine or bromine and
$X^5$ represents hydrogen or chlorine, and in addition,
$X^1$ and $X^3$ together can represent a C-C bond, and also
$X^2$ and $X^4$ together can represent a C-C bond, or
$X^1$ and $X^2$ together represent oxygen or the grouping =N−Y,
in which
Y represents hydroxyl and the radicals

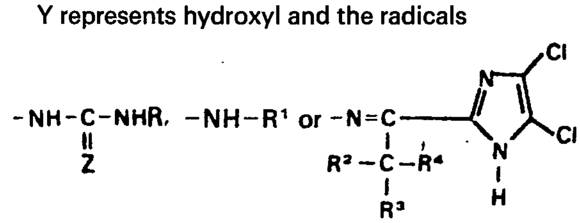

in which:
R represents hydrogen or alkyl with 1 to 4 carbon atoms,
$R^1$ represents phenyl or naphthyl optionally substituted by fluorine, chlorine, bromine, alkyl with 1 or 2 carbon atoms, alkoxy with 1 or 2 carbon atoms, trichloromethyl, trifluoromethyl and/or nitro,
Z represents oxygen or sulphur and
$R^2$, $R^3$ and $R^4$ independently of one another represent hydrogen or chlorine,
but wherein $X^1$, $X^2$, $X^3$, $X^4$ and $X^5$ do not simultaneously represent hydrogen,
characterised in that
a) 2-ethylimidazole of the formula II

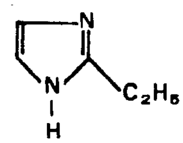
(II)

or its hydrochloride is reacted with up to 7 mols of chlorine per mol of 2-ethylimidazole in a solvent which is inert under the reaction conditions, or
b) a 4,5-dichloroimidazole derivative of the formula III

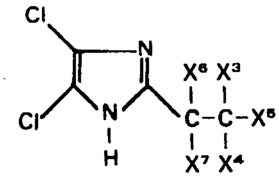
(III)

in which
$X^6$ represents chlorine or bromine,
$X^7$ represents chlorine or bromine and
$X^3$, $X^4$ and $X^5$ have the meaning indicated above, but wherein $X^3$, $X^4$ and $X^5$ do not simultaneously represent hydrogen,
α) is hydrolysed with water, or
β) is reacted with at least the stoichiometrically required amount of a dehalogenating agent, if appropriate in the presence of a diluent, or
γ) is reacted with at least the stoichiometrically required amount of a metal hydride customary for hydrogenation reactions, in the presence of a diluent, or
c) a 4,5-dichloroimidazole derivative of the formula IV

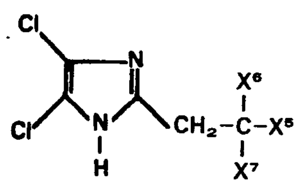
(IV)

in which
$X^5$, $X^6$ and $X^7$ have the meaning indicated above, is reacted with an agent which splits off hydrogen chloride, in the presence of a diluent, or
d) 2,4,5-trichloro-2-(1,1-dichloroethyl)-2H-imidazole of the formula V

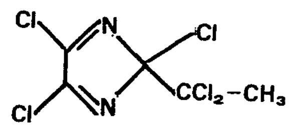
(V)

α) is reacted with a reducing agent, in presence of a diluent, or
β) is reacted with an excess of at least 3 mols of an alkali metal iodide or with an excess of at least 1.5 mols of an alkaline earth metal iodide, in the presence of a lower aliphatic ketone as the diluent, or

γ) is reacted with an at least equimolar amount of an alkali metal bromide or with an at least equivalent amount of an alkaline earth metal bromide, in the presence of a lower aliphatic nitrile or of a lower aliphatic ketone as the diluent, or

e) a 4,5-dichloroimidazole derivative of the formula VI

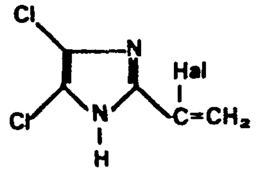

(VI)

in which

Hal represents chlorine or iodine,

α) is reacted with an agent which splits off hydrogen halide, if appropriate in the presence of a diluent, or

β) is hydrolysed with water, in the presence of a base or of a basic salt, or

f) a 4,5-dichloroimidazole derivative of the formula VII

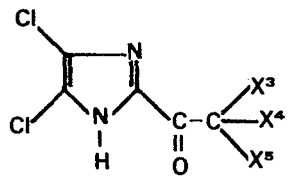

(VII)

in which

$X^3$, $X^4$ and $X^5$ have the meaning indicated above, is reacted with a compound of the formula VIII

$$H_2N-Y' \qquad (VIII)$$

in which:

Y' represents amino, hydroxyl or the radicals

$$-NH-\underset{\underset{Z}{\|}}{C}-NHR$$

or $-NHR^1$

in which

Z represents oxygen or sulphur,

R represents hydrogen or alkyl with 1 to 4 carbon atoms and

$R^1$ represents phenyl or naphthyl optionally substituted by fluorine, chlorine, bromine, alkyl with 1 or 2 carbon atoms, alkoxy with 1 or 2 carbon atoms, trichloromethyl, trifluoromethyl and/or nitro,

in the presence of a diluent, or

g) 4,5-dichloro-2-ethinylimidazole of the formula IX

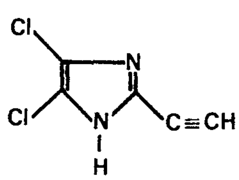

(IX)

is reacted with a chlorinating or brominating agent, in the presence of a solvent which is inert under the reaction conditions, or

h) 4,5-dichloroimidazole derivatives of the formula X

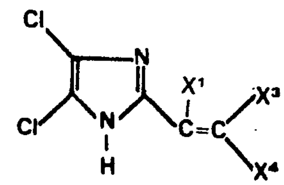

(X)

in which

$X^1$, $X^3$ and $X^4$ have the meaning indicated above, are reacted with a chlorinating or brominating agent, in the presence of a solvent which is inert under the reaction conditions, or

i) 4,5-dichloroimidazole derivatives of the formula XI

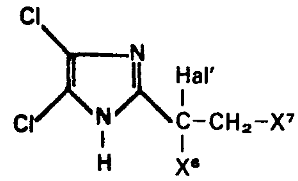

(XI)

in which

$X^6$ and $X^7$ have the meaning indicated above and

Hal' represents chlorine, bromine or iodine,

are dehydrohalogenated under the influence of heat, in the presence of a lower aliphatic nitrile.

3. Herbicidal agents, characterised in that they contain at least one 4,5-dichloroimidazole derivative of the formula I according to claim 1.

4. Use of 4,5-dichloroimidazole derivates of the formula I according to claim 1 for combating weeds.

5. Process for the preparation of herbicidal agents, characterised in that 4,5-dichloroimidazole derivatives of the formula I according to claim 1 are mixed with extenders and/or surface-active substances.

## Revendications

1. Dérivés de 4,5-dichlorimidazoles de formule I:

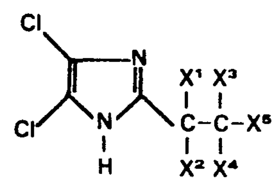

(I)

dans laquelle

$X^1$ représente un atome d'hydrogène, un atome de chlore, un atome de brome ou un atome d'iode,

$X^2$, $X^3$ et $X^4$ représente chacun un atome d'hydrogène, un atome de chlore ou un atome de brome,

$X^5$ représente un atome d'hydrogène ou un atome de chlore, et, en outre

$X^1$ et $X^3$ ensemble peuvent représenter une liaison C-C, et

$X^2$ et $X^4$ ensemble peuvent représenter une liaison C-C, ou

$X^1$ et $X^2$ ensemble représentent un atome d'oxygène ou le groupement $=N-Y$ dans lequel

Y représente un groupe hydroxy, le groupe

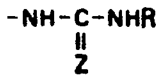

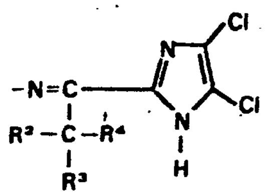

le groupe $-NH-R^1$ ou le groupe

où

R représente un atome d'hydrogène ou un groupe alkyle contenant 1 à 4 atomes de carbone,

$R^1$ représente un groupe naphtyle ou un groupe phényle éventuellement substitué par un atome de fluor, un atome de chlore, un atome de brome, un groupe alkyle contenant 1 ou 2 atomes de carbone, un groupe alcoxy contenant 1 ou 2 atomes de carbone, un groupe trichlorométhyle, un groupe trifluorométhyle et/ou par un groupe nitro,

Z représente un atome d'oxygène ou un atome de soufre, et

$R^2$, $R^3$ et $R^4$ représentent chacun indépendamment l'un de l'autre un atome d'hydrogène ou un atome de chlore,

$X^1$, $X^2$, $X^3$, $X^4$ et $X^5$ ne pouvant cependant pas représenter simultanément des atomes d'hydrogène.

2. Procédé de préparation de dérivés de 4,5-dichlorimidazoles de formule I:

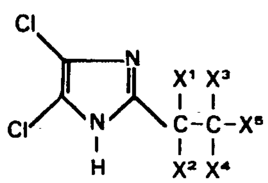

(I)

dans laquelle

$X^1$ représente un atome d'hydrogène, un atome de chlore, un atome de brome ou un atome d'iode,

$X^2$, $X^3$ et $X^4$ représentent chacun un atome d'hydrogène, un atome de chlore ou un atome de brome,

$X^5$ représente un atome d'hydrogène ou un atome de chlore et, en outre,

$X^1$ et $X^3$ ensemble peuvent représenter une liaison C-C, et

$X^2$ et $X^4$ ensemble peuvent représenter une liaison C-C, ou

$X^1$ et $X^2$ ensemble représentent un atome d'oxygène ou le groupement $=N-Y$ où

Y représente un groupe hydroxy, le groupe

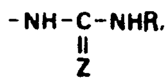

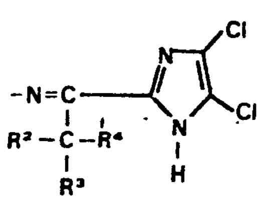

le groupe $-NH-R^1$ ou le groupe

où

R représente un atome d'hydrogène ou un groupe alkyle contenant 1 à 4 atomes de carbone,

$R^1$ représente un groupe naphtyle ou un groupe phényle éventuellement substitué par un atome de fluor, un atome de chlore, un atome de brome, un groupe alkyle contenant 1 ou 2 atomes de carbone, un groupe alcoxy contenant 1 ou 2 atomes de carbone, un groupe trichlorométhyle, un groupe trifluorométhyle et/ou par un groupe nitro,

Z représente un atome d'oxygène ou un atome de soufre, et

$R^2$, $R^3$ et $R^4$ représentent chacun indépendamment l'un de l'autre un atome d'hydrogène ou un atome de chlore,

$X^1$, $X^2$, $X^3$, $X^4$ et $X^5$ ne pouvant cependant pas représenter simultanément des atomes d'hydrogène caractérisé en ce que:

a) on fait réagir le 2-éthylimidazole de formule II:

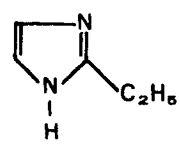

(II)

ou son chlorhydrate avec jusqu'à 7 mol de chlore/mol de 2-éthylimidazole dans un solvant inerte dans les conditions réactionnelles, ou

b) on hydrolyse un dérivé de 4,5-dichlorimidazole de formule III:

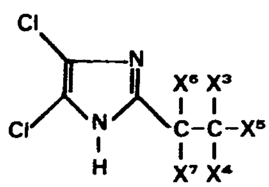

(III)

dans laquelle

$X^6$ représente un atome de chlore ou un atome de brome,

$X^7$ représente un atome de chlore ou un atome de brome,

et

$X^3$, $X^4$ et $X^5$ ont les significations indiquées ci-dessus, $X^3$, $X^4$ et $X^5$ ne pouvant toutefois pas représenter simultanément des atomes d'hydrogène,

α) avec de l'eau,

ou

β) on fait réagir ce dérivé avec au moins la quantité stœchiométriquement nécessaire d'un agent de déshalogénation éventuellement en présence d'un diluant,

ou

γ) on fait réagir ce dérivé avec au moins la quantité stœchiométriquement nécessaire d'un hydrure métallique habituellement utilisé pour l'hydrogénation, en présence d'un diluant,

ou

c) on fait réagir un dérivé de 4,5-dichlorimidazole de formule IV:

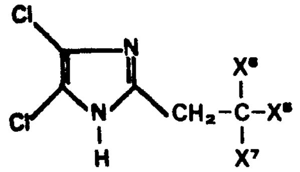

(IV)

dans laquelle

$X^5$, $X^6$ et $X^7$ ont les significations indiquées ci-dessus,

avec un agent générateur de chlorure d'hydrogène, en présence d'un diluant, ou

d) on fait réagir le 2,4,5-trichloro-2-(1,1-dichloréthyl)-2H-imidazole de formule V:

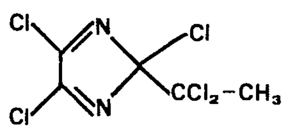

(V)

α) avec un agent réducteur en présence d'un diluant, ou

β) on le fait réagir avec un excès au moins 3 molaires d'un iodure d'un métal alcalin ou avec un excès au moins 1,5 molaire d'un iodure d'un métal alcalino-terreux en présence d'une cétone aliphatique inférieure comme diluant, ou

γ) on le fait réagir avec une quantité au moins équimolaire d'un bromure d'un métal alcalin ou avec une quantité au moins équivalente d'un bromure d'un métal alcalino-terreux en présence d'un nitrile aliphatique inférieur ou d'une cétone aliphatique inférieure comme diluant, ou

e) on fait réagir un **dérivé** de 4,5-dichlorimidazole de formule VI:

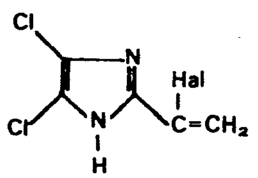

(VI)

dans laquelle

Hal représente un atome de chlore ou un atome d'iode,

α) avec un agent générateur d'un hydracide halogéné, éventuellement en présence d'un diluant, ou

β) on hydrolyse ce dérivé avec de l'eau en présence d'une base ou d'un sel basique, ou

f) on fait réagir un dérivé de 4,5-dichlorimidazole de formule VII:

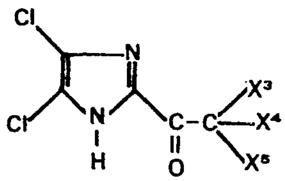

(VII)

dans laquelle

$X^3$, $X^4$ et $X^5$ ont les significations indiquées ci-dessus,

avec un composé de formule VIII:

$H_2N-Y'$ (VIII)

dans laquelle

Y' représente un groupe amino, un groupe hydroxy, un groupe

$$-NH-\underset{\underset{Z}{\|}}{C}-NHR$$

ou un groupe $-NHR^1$

où

Z représente un atome d'oxygène ou un atome de soufre,

R représente un atome d'hydrogène ou un groupe alkyle contenant 1 à 4 atomes de carbone, et

$R^1$ représente un groupe naphtyle ou un groupe phényle éventuellement substitué par un atome de fluor, un atome de chlore, un atome de brome, un groupe alkyle contenant 1 ou 2 atomes de carbone, un groupe alcoxy contenant 1 ou 2 atomes de carbone, un groupe trichlorométhyle, un groupe trifluorométhyle et/ou par un groupe nitro, en présence d'un diluant, ou

g) on fait réagir le 4,5-dichloro-2-éthinyl-imidazole de formule IX:

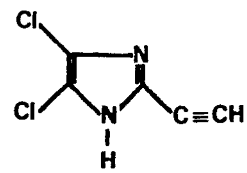

(IX)

avec un agent de chloration ou un agent de bromation en présence d'un solvant inerte dans les conditions réactionnelles, ou

h) on fait réagir des dérivés de 4,5-dichlorimidazoles de formule X:

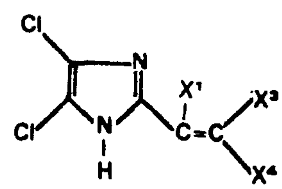

(X)

dans laquelle:

$X^1$, $X^3$ et $X^4$ ont les significations indiquées ci-dessus,

avec un agent de chloration ou un agent de bromation en présence d'un solvant inerte dans les conditions réactionnelles, ou

i) on soumet des dérivés de 4,5-dichlorimidazoles de formule XI:

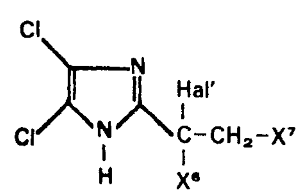

(XI)

dans laquelle

$X^6$ et $X^7$ ont les significations indiquées ci-dessus, et

Hal' représente un atome de chlore, un atome de brome ou un atome d'iode,

à une déshydrohalogénation thermique en présence d'un nitrile aliphatique inférieur.

3. Agents herbicides, caractérisés en ce qu'ils contiennent au moins un dérivé de 4,5-dichlorimidazole de formule I suivant la revendication 1.

4. Utilisation de dérivés de 4,5-dichlorimidazoles de formule I suivant la revendication 1 pour combattre les plantes adventices.

5. Procédé de préparation d'agents herbicides, caractérisé en ce qu'on mélange des dérivés de 4,5-dichlorimidazoles de formule I suivant la revendication 1 avec des diluants et/ou des substances tensio-actives.